# EUROPEAN PATENT APPLICATION

(11) **EP 3 851 517 A1**
(43) Date of publication of application: **21.07.2021**
(21) Application number: 19858845.1
(22) Date of filing: 10.09.2019
(51) Int. Cl.: C12N 1/00

(54) **COMPOSITION FOR INHIBITING TRYPSIN ACTIVITY, CONTAINING, AS ACTIVE INGREDIENT, BACTERIUM BELONGING TO GENUS PARAPREVOTELLA**

(30) Priority: 10.09.2018 US 201862728908 P; 18.01.2019 US 201962794145 P
(71) Applicant: RIKEN, Wako-shi Saitama 351-0198 (JP)
(72) Inventor: HONDA Kenya, Wako-shi, Saitama 351-0198 (JP); NARUSHIMA Seiko, Wako-shi, Saitama 351-0198 (JP); WATANABE Eiichiro, Wako-shi, Saitama 351-0198 (JP); OHARA Osamu, Wako-shi, Saitama 351-0198 (JP); KAWASHIMA Yusuke, Wako-shi, Saitama 351-0198 (JP); LI Youxian, Wako-shi, Saitama 351-0198 (JP)
(74) Representative: J A Kemp LLP
(86) International application number: PCT/JP2019/035574
(87) International publication number: WO 2020/054728

(57) **Abstract**

The present invention provides a composition for suppressing trypsin activity, comprising: a bacterium belonging to a genus Paraprevotella as an active ingredient.

## Description

### [Technical Field]

The present invention relates to a composition for suppressing trypsin activity, which contains a bacterium belonging to a genus Paraprevotella as an active ingredient. Also, the present application claims priority to U.S. Patent Provisional Application No. 62/728908 (filed on September 10, 2018) and U.S. Patent Provisional Application No. 62/794145 (filed on January 18, 2019), the disclosure of which is incorporated herein by reference.

### [Background Art]

It is thought that about 100 trillion intestinal bacteria inhabit the human digestive tract. This means that far more intestinal bacteria coexist with us than about 37 trillion human somatic cells, forming an intestinal microbiota. Since the proposal of the intestinal microbiology, various studies have been conducted. In fact, recent experiments with germ-free animals have revealed that the intestinal microbiota has various effects on the maturation of the host immune system and the functions of the host. For example, intestinal bacteria contribute to the suppression of colonization and growth of pathogenic microbes by competition of the pathogenic microbes with niches. In addition, the intestinal bacteria of an obese human contain less Bacteroidetes, and germ-free mice transplanted with the feces thereof increase in body fat.

In addition, with the spread of gnotobiotic technology, detailed studies on the effects of individual bacterial species from all intestinal bacteria on the host immune system are beginning to be reported. For example, 17 bacterial strains of the Clostridia group isolated from healthy individuals induce regulatory T cells in the large intestine of a mouse and alleviate host inflammation (NPL 1). In addition, segmented filamentous bacteria (SFB) inhabiting the small intestine of a mouse adhere to the small intestinal epithelium to strongly induce Thl7 cells (NPL 2). Furthermore, Klebsiella pneumoniae isolated from the saliva of a Crohn's disease (CD) patient ectopically colonizes the large intestine of a mouse, thereby strongly inducing Th1 cells and causing colitis (NPL 3). As above, while there are many studies on intestinal bacteria and host immune systems, there are a small number of detailed studies focusing on individual intestinal bacterial species and host-derived proteins present in feces (NPLs 4 to 7).

In addition, among host-derived proteins, trypsin is known as one of the proteolytic enzymes secreted from the pancreas. Recently, it has been suggested that residual active trypsin beyond the large intestine is involved in the onset and exacerbation of various diseases. Specifically, it has been reported that trypsin remains in the feces of patients with inflammatory bowel disease (IBD), particularly CD patients, while maintaining its activity (NPLs 8 and 9). In addition, it has been suggested that the genus Bacteroides, which is known as a major component of human intestinal bacteria, decreases in IBD patients (NPL 9) and that certain species of bacteria constituting the genus Bacteroides are involved in the inactivation of trypsin in feces (NPL 10), but there are few detailed studies.

In view of the above situation, it is expected that the isolation of bacteria capable of suppressing trypsin activity would lead to the development of bacterial therapy that suppresses trypsin activity as a new therapeutic strategy for IBD, but no such bacteria have been isolated yet.

### [Citation List]

### [Non Patent Literature]

[NPL 1] Atarashi, K. et al. Nature 500, 232-236, doi: 10.1038/nature12331 (2013).
[NPL 2] Atarashi, K. et al. Cell 163, 367-380, doi: 10.1016/j.cell.2015.08.058 (2015).
[NPL 3] Atarashi, K. et al. Science 358, 359-365, doi: 10.1126/science.aan4526 (2017).
[NPL 4] Palm, N. W. et al. Cell 158, 1000-1010, doi: 10.1016/j.cell.2014.08.006 (2014).
[NPL 5] Kawamoto, S. et al. Immunity 41, 152-165, doi: 10.1016/j.immuni.2014.05.016 (2014).
[NPL 6] Planer, J. D. et al. Nature 534, 263-266, doi: 10.1038/nature17940 (2016).
[NPL 7] Bunker, J. J. et al. Immunity 43, 541-553, doi: 10.1016/j.immuni.2015.08.007 (2015).
[NPL 8] van de Merwe, J. P. & Mol, G. J. Digestion 24, 1-4, doi: 10.1159/000198767 (1982).
[NPL 9] Midtvedt, T. et al. PLoS One 8, e66074, doi: 10.1371/journal.pone.0066074 (2013).
[NPL 10] Ramare, F., Hautefort, I., Verhe, F., Raibaud, P. & Iovanna,. Appl Environ Microbiol 62, 1434-1436 (1996).

### [Summary of Invention]

### [Technical Problem]

The present invention has been made in view of the above-mentioned problems of the related art, and an object thereof is to isolate a bacterium capable of suppressing trypsin activity, and to provide a composition for suppressing trypsin activity, which contains the bacterium as an active ingredient.

### [Solution to Problem]

The present inventors have made intensive studies to achieve the above object, and as a result have identified 713 host-derived proteins by performing comprehensive protein analysis (proteome analysis) on the cecal contents of SPF mice and germ-free mice (GF mice). Compared to SPF mice, 45 proteins were significantly more abundant in the cecal contents of GF mice. Of these, attention was paid to the proteolytic enzyme trypsin (anionic trypsin-2: PRSS2), which showed a particularly significant difference in abundance.

Trypsin is a gastrointestinal enzyme secreted from the exocrine glands of the pancreas, a potent proteolytic enzyme, and has been suggested to be associated with IBD of unknown cause. With the above in mind, the expression level and secretion amount of the trypsin precursor trypsinogen in pancreatic tissues were examined, but no difference was observed between SPF mice and GF mice. On the other hand, regarding the trypsin activity in the intestinal contents, no significant difference was observed in the small intestine, but a significant decrease in activity was observed in SPF mice beyond the cecum. From the above, it was considered that the intestinal bacteria are factors that reduce the trypsin activity beyond the cecum.

Next, when fecal samples of 6 healthy volunteers were inoculated to GF mice to prepare human microbiota-colonized mice, the fecal trypsin activity was different for each sample group. Thus, the bacterial strains for reducing fecal trypsin activity were identified. The cecal contents of the human microbiota-colonized mice with the lowest trypsin activity were inoculated to other GF mice, and fecal trypsin activity was evaluated while inoculating antibiotics having various antibacterial spectra. As a result, a significant decrease in fecal trypsin activity was observed in the ampicillin-inoculated mouse group. Among them, for the mouse having the lowest trypsin activity, the bacteria contained in the cecal contents were cultured under anaerobic conditions to isolate 35 strains of the bacteria responsible for reducing trypsin activity. Furthermore, the responsible bacteria for reducing trypsin activity were narrowed down. Bacterial strains were narrowed down based on the Spearman rank correlation analysis of the relative occupancy of the intestinal bacteria and the trypsin activity value in the feces of the mouse inoculated with antibiotics having various antibacterial spectra described above. It has been clarified that the 3 bacterial strains Paraprevotella clara (P. clara), Parabacteroides merdae (P. merdae), and Bacteroides uniformis (B. uniformis) reduce trypsin activity. In addition, it has been finally found that P. clara monobacterium is the responsible bacterium in suppressing trypsin activity.

Furthermore, as a result of also evaluating other bacteria belonging to the genus Paraprevotella (P. xylaniphila), it has been clarified that trypsin activity can be suppressed in the same manner as in the case of P. clara.

The present invention has been created based on such findings, and relates to a composition for suppressing trypsin activity, which contains a bacterium belonging to a genus Paraprevotella as an active ingredient, and more specifically provides the following.
<1> A composition for suppressing trypsin activity, comprising: a bacterium belonging to a genus Paraprevotella as an active ingredient.
<2> The composition according to <1>, wherein the bacterium belonging to the genus Paraprevotella is at least one bacterium selected from the group consisting of Paraprevotella clara and Paraprevotella xylaniphila.
<3> The composition according to <1>, wherein the bacterium belonging to the genus Paraprevotella is at least one bacterium having a DNA composed of a base sequence set forth in any of SEQ ID NOs : 1 to 3 or a base sequence having at least 90% identity to the base sequence.
<4> The composition according to <1>, wherein the bacterium belonging to the genus Paraprevotella is at least one bacterial strain selected from the group consisting of a bacterial strain belonging to Paraprevotella clara specified by accession number NITE BP-02775 (Paraprevotella clara 1C4 strain), a Paraprevotella clara JCM 14859^{T} strain, and a Paraprevotella xylaniphila JCM 14860^{T} strain.
<5> The composition according to any one of <1> to <4>, further comprising: at least one bacterium selected from the group consisting of Parabacteroides merdae and Bacteroides uniformis.
<6> The composition according to <5>, wherein the Parabacteroides merdae is at least one bacterium having a DNA composed of a base sequence set forth in SEQ ID NO: 4 or a base sequence having at least 90% identity to the base sequence, and the Bacteroides uniformis is at least one bacterium having a DNA composed of a base sequence set forth in SEQ ID NO: 5 or a base sequence having at least 90% identity to the base sequence.
<7> The composition according to <5>, wherein the Parabacteroides merdae is a bacterial strain belonging to Parabacteroides merdae specified by accession number NITE BP-02776 (Parabacteroides merdae 1D4 strain), and the Bacteroides uniformis is a bacterial strain belonging to Bacteroides uniformis specified by accession number NITE BP-02777 (Bacteroides uniformis 3H3 strain).
<8> A bacterial strain belonging to Paraprevotella clara specified by accession number NITE BP-02775 (Paraprevotella clara 1C4 strain).
<9> A bacterial strain belonging to Parabacteroides merdae specified by accession number NITE BP-02776 (Parabacteroides merdae 1D4 strain).
<10> A bacterial strain belonging to Bacteroides uniformis specified by accession number NITE BP-02777 (Bacteroides uniformis 3H3 strain).
   In addition, active trypsin remaining in the large intestine has long been suggested to be involved in the onset and exacerbation of symptoms of IBD known as ulcerative colitis and Crohn's disease. In light of the above, the present inventors verified the alleviation of inflammation based on the effect of suppressing the trypsin activity of the above 3 bacterial strains using a colitis model mouse. In a model in which an IL-10 gene-deficient mouse is infected with Enterobacter aerogenes to induce colitis, inoculation of the above 3 bacterial strains tended to suppress the onset thereof. In addition, the onset of colitis was significantly suppressed in a DSS-induced colitis model.
   Therefore, based on such findings, the present invention also provides pharmaceutical compositions and methods for treating, ameliorating, or preventing the following diseases caused by trypsin activity.
<11> A pharmaceutical composition for treating, ameliorating, or preventing a disease caused by trypsin activity, comprising: at least one bacterium selected from the group consisting of a bacterium belonging to a genus Paraprevotella, Parabacteroides merdae, and Bacteroides uniformis as an active ingredient.
<12> The pharmaceutical composition according to <11>, wherein the bacterium belonging to the genus Paraprevotella is at least one bacterium selected from the group consisting of Paraprevotella clara and Paraprevotella xylaniphila.
<13> The pharmaceutical composition according to <11>, wherein the bacterium belonging to the genus Paraprevotella is at least one bacterium having a DNA composed of a base sequence set forth in any of SEQ ID NOs: 1 to 3 or a base sequence having at least 90% identity to the base sequence.
<14> The pharmaceutical composition according to <11>, wherein the bacterium belonging to the genus Paraprevotella is at least one bacterial strain selected from the group consisting of a bacterial strain belonging to Paraprevotella clara specified by accession number NITE BP-02775 (Paraprevotella clara 1C4 strain), a Paraprevotella clara JCM 14859^{T} strain, and a Paraprevotella xylaniphila JCM 14860^{T} strain.
<15> The pharmaceutical composition according to any one of <11> to <14>, wherein the Parabacteroides merdae is at least one bacterium having a DNA composed of a base sequence set forth in SEQ ID NO: 4 or a base sequence having at least 90% identity to the base sequence.
<16> The pharmaceutical composition according to any one of <11> to <14>, wherein the Parabacteroides merdae is a bacterial strain belonging to Parabacteroides merdae specified by accession number NITE BP-02776 (Parabacteroides merdae 1D4 strain).
<17> The pharmaceutical composition according to any one of <11> to <16>, wherein the Bacteroides uniformis is at least one bacterium having a DNA composed of a base sequence set forth in SEQ ID NO: 5 or a base sequence having at least 90% identity to the base sequence.
<18> The pharmaceutical composition according to any one of <11> to <16>, wherein the Bacteroides uniformis is a bacterial strain belonging to Bacteroides uniformis specified by accession number NITE BP-02777 (Bacteroides uniformis 3H3 strain).
<19> The pharmaceutical composition according to any one of <11> to <18>, wherein the disease caused by trypsin activity is inflammatory bowel disease.
<20> The pharmaceutical composition according to <19>, wherein the inflammatory bowel disease is any one of ulcerative colitis (UC) and Crohn's disease (CD).
<21> A method for treating, ameliorating, or preventing a disease caused by trypsin activity in a target by allowing the target to ingest at least one bacterium selected from the group consisting of a bacterium belonging to a genus Paraprevotella, Parabacteroides merdae, and Bacteroides uniformis.
<22> The method according to <21>, wherein the bacterium belonging to the genus Paraprevotella is at least one bacterium selected from the group consisting of Paraprevotella clara and Paraprevotella xylaniphila.
<23> The method according to <21>, wherein the bacterium belonging to the genus Paraprevotella is at least one bacterium having a DNA composed of a base sequence set forth in any of SEQ ID NOs: 1 to 3 or a base sequence having at least 90% identity to the base sequence.
<24> The method according to <21>, wherein the bacterium belonging to the genus Paraprevotella is at least one bacterial strain selected from the group consisting of a bacterial strain belonging to Paraprevotella clara specified by accession number NITE BP-02775 (Paraprevotella clara 1C4 strain), a Paraprevotella clara JCM 14859^{T} strain, and a Paraprevotella xylaniphila JCM 14860^{T} strain.
<25> The method according to any one of <21> to <24>, wherein the Parabacteroides merdae is at least one bacterium having a DNA composed of a base sequence set forth in SEQ ID NO: 4 or a base sequence having at least 90% identity to the base sequence.
<26> The method according to any one of <21> to <24>, wherein the Parabacteroides merdae is a bacterial strain belonging to Parabacteroides merdae specified by accession number NITE BP-02776 (Parabacteroides merdae 1D4 strain).
<27> The method according to any one of <21> to <26>, wherein the Bacteroides uniformis is at least one bacterium having a DNA composed of a base sequence set forth in SEQ ID NO: 5 or a base sequence having at least 90% identity to the base sequence.
<28> The method according to any one of <21> to <26>, wherein the Bacteroides uniformis is a bacterial strain belonging to Bacteroides uniformis specified by accession number NITE BP-02777 (Bacteroides uniformis 3H3 strain).
<29> The method according to any one of <21> to <28>, wherein the disease caused by trypsin activity is inflammatory bowel disease.
<30> The method according to <29>, wherein the inflammatory bowel disease is any one of ulcerative colitis and Crohn's disease.

### [Advantageous Effects of Invention]

According to the present invention, it is possible to suppress trypsin activity. In particular, it becomes possible to suppress the intestinal trypsin activity.

### [Brief Description of Drawings]

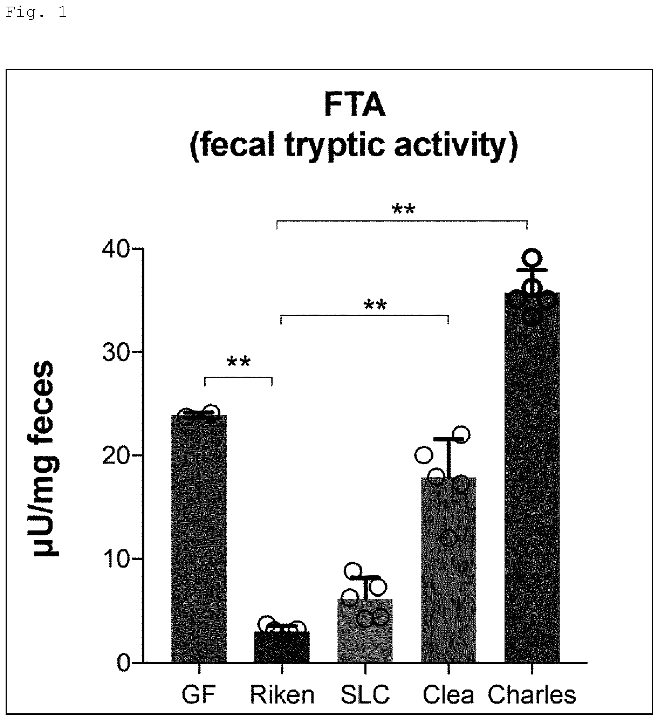

[Fig. 1] Fig. 1 is a graph showing the results of evaluating the trypsin activity in the cecal contents (feces) of SPF mice derived from different breeding facilities. In the figure, "GF," "Riken," "SLC," "Clea," and "Charles" described on the horizontal axis indicate a germ-free mouse (GF mouse), an SPF mouse maintained by RIKEN, an SPF mouse maintained by Japan SLC, Inc., an SPF mouse maintained by CLEA Japan, Inc., and an SPF mouse maintained by Charles River Laboratories Japan, Inc., and the vertical axis indicates the trypsin activity in each cecal content.

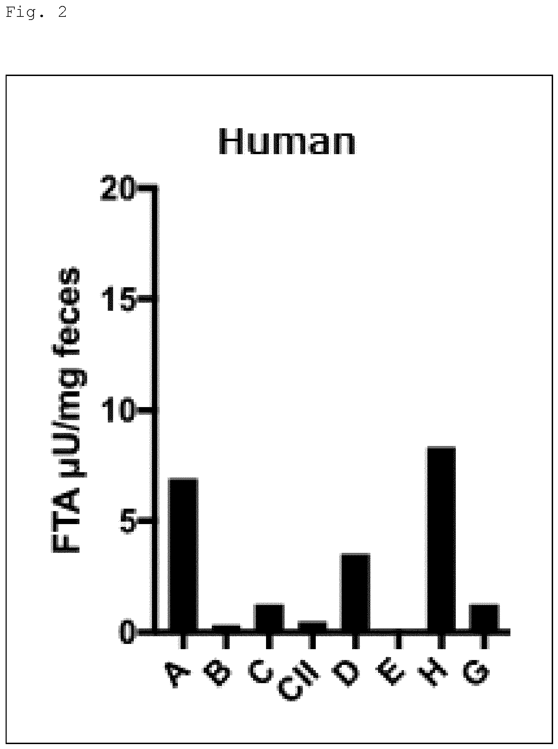

[Fig. 2] Fig. 2 is a graph showing the results of evaluating the fecal trypsin activity of healthy Japanese volunteers (A, B, C, CII, D, E, H, and G). The horizontal axis in the figure indicates each healthy volunteer, and the vertical axis indicates fecal trypsin activity. Note that the providers of the feces C and CII are the same healthy volunteer.

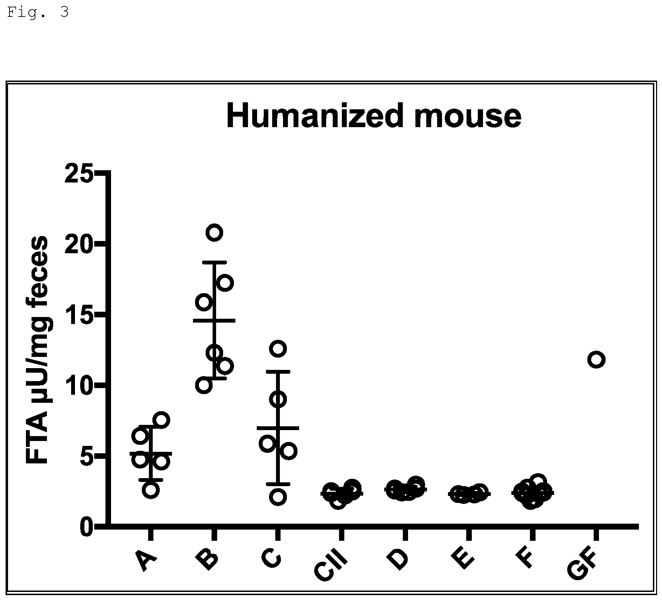

[Fig. 3] Fig. 3 is a graph showing the results of evaluating fecal trypsin activity by orally inoculating the feces of the healthy Japanese volunteers (A to F) into the stomach of GF mice to prepare human microbiota-transplanted mice (A to F). The horizontal axis in the figure indicates human microbiota-transplanted mice and GF mice, and the vertical axis indicates fecal trypsin activity.

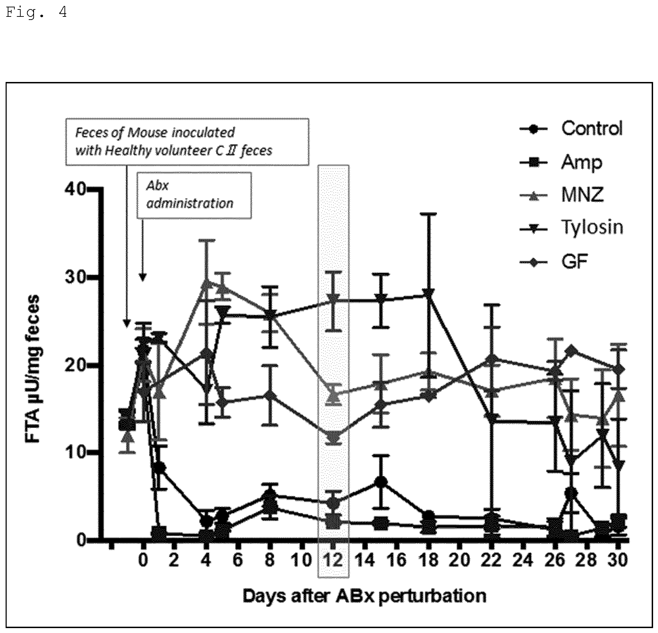

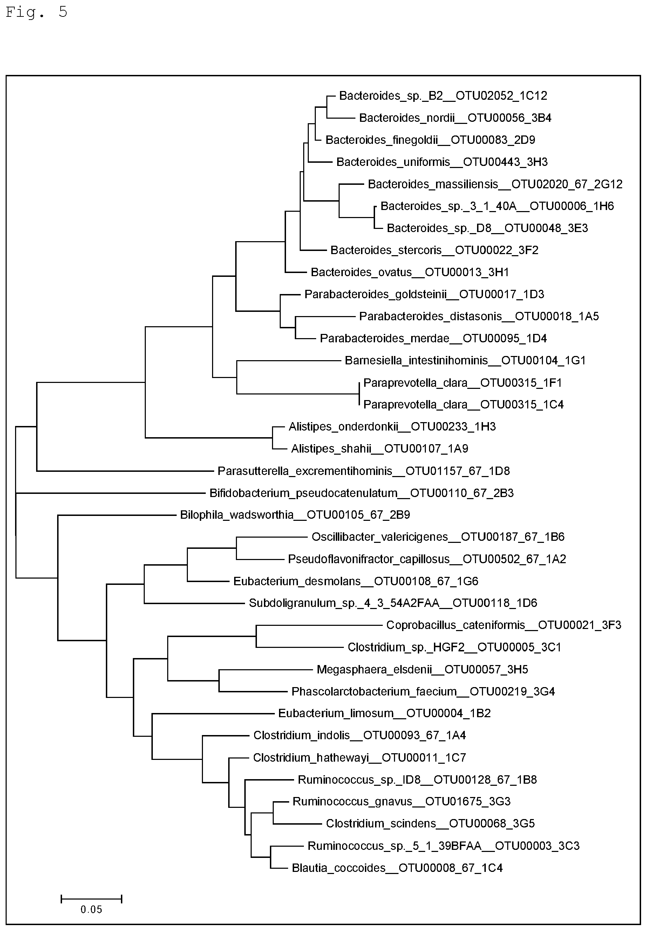

[Fig. 4] Fig. 4 is a graph showing the results of evaluating the fecal trypsin activity over time on the GF mice that were orally inoculated with the feces CII of the healthy volunteer into the stomach, and 24 hours later were inoculated by free water intake with the antibiotics (Abx) ampicillin (Amp), tylosin, or metronidazole (MNZ). The horizontal axis in the figure indicates the number of days after inoculation of the antibiotics, and the vertical axis indicates fecal trypsin activity. Note that the figure also shows the results of evaluating the GF mice (control) inoculated with only the feces CII of the healthy volunteer (no antibiotics inoculated) and the GF mice (GF) inoculated with neither feces nor antibiotics. The number of individuals evaluated is as follows. Control:Amp:MNZ:Tylosin:GF = 4:5:5:4:2. Since the tendency of the activity transition at the time of Amp inoculation was the same as that of the antibiotic non-inoculated group (control), the bacterial group contributing to the decrease in activity was presumed to be Amp-resistant bacteria. Based on the above, the bacteria were isolated and cultured from the fecal samples of the Amp-inoculated group to obtain a total of 35 bacterial strains shown in Fig. 5 below. Subsequently, the microbiota of each individual on the 12th day from the start of antibiotic inoculation was derived by meta-16S rDNA analysis. Furthermore, a correlation analysis was performed on the relative occupancy of each of the bacterial species constituting the microbiota and the trypsin activity value of that individual, to thereby estimate the bacterial species contributing to the decrease in trypsin activity.
[Fig. 5] Fig. 5 shows a phylogenetic tree based on the homology of 16S rDNA sequences of a total of 35 bacterial strains isolated from the fecal samples of the Amp-inoculated group shown in Fig. 4. The notation in the figure shows, in order from the left, the name of bacterial species assigned, the representative OTU No. of 16S rDNA analysis, and the named isolated bacterial strain.

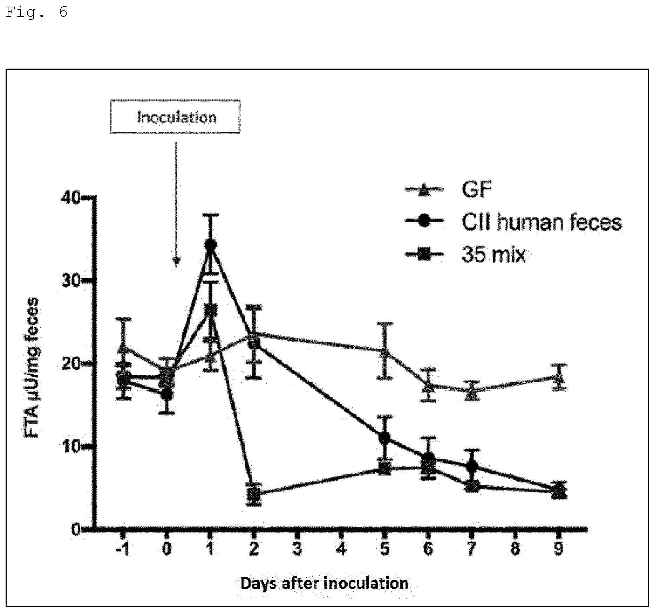

[Fig. 6] Fig. 6 is a graph showing the results of evaluating over time the fecal trypsin activity of mice colonized with the isolated 35 bacteria. The horizontal axis in the figure indicates the number of days after inoculation of the bacteria or feces, and the vertical axis indicates fecal trypsin activity. The isolated 35 bacteria were inoculated to GF mice ("35 mix" in the figure) to evaluate the intestinal trypsin activity, and the same or better effects were observed as compared with the transplantation of bacteria-derived feces ("CII human faces" in the figure).

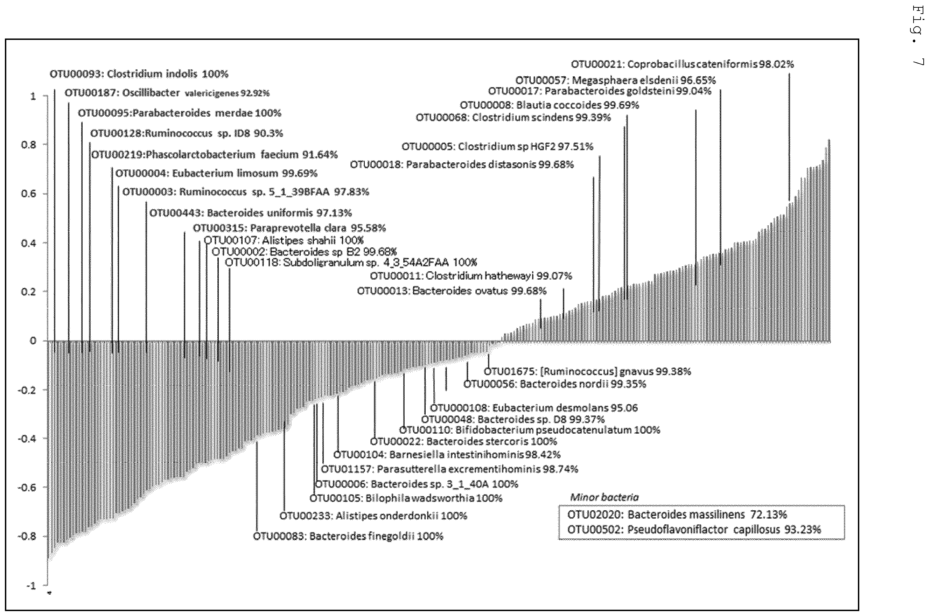

[Fig. 7] Fig. 7 is a diagram showing Spearman's correlation of microbiota and trypsin activity. More specifically, it is a diagram showing the results of expressing the bacterial species whose uncorrelated test P-value satisfies P < 0.05 in the ascending order of the correlation coefficient ρ. Spearman's correlation was used to estimate the bacterial strains whose abundance was correlated with the fecal trypsin activity value of each individual. Of these, attention was paid to 9 bacterial strains with a strong negative correlation (9 bacteria from the left).

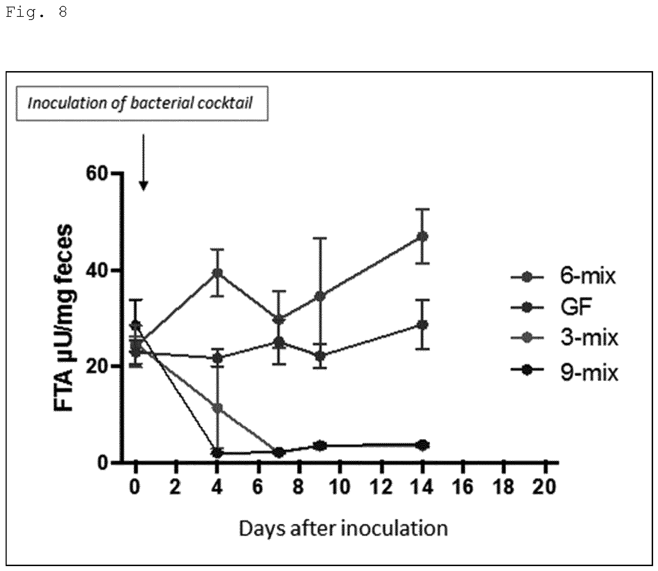

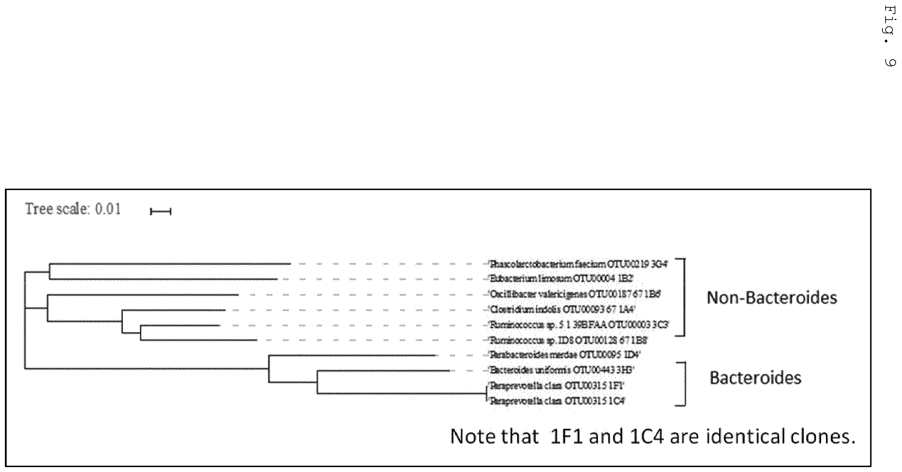

[Fig. 8] Fig. 8 is a graph showing the results of evaluating over time the fecal trypsin activity of mice colonized with the 9 bacterial strains (9-mix) observed to have a strong negative correlation in Spearman's correlation shown in Fig. 7. Further, the mice colonized with the 6 bacterial strains (6-mix) or 3 bacterial strains (3-mix) shown in Fig. 9 below were also evaluated in the same manner. The horizontal axis in the figure shows the number of days since each bacterial cocktail was inoculated, and the vertical axis shows the fecal trypsin activity.
[Fig. 9] Fig. 9 shows a phylogenetic tree of the 9 bacterial strains observed to have a strong negative correlation in Spearman's correlation shown in Fig. 7. As shown in the figure, the 9 bacterial strains are divided into 3 bacterial strains belonging to the genus Bacteroides ("Bacteroides" in the figure) and 6 bacterial strains other than the genus Bacteroides ("Non-Bacteroides" in the figure).

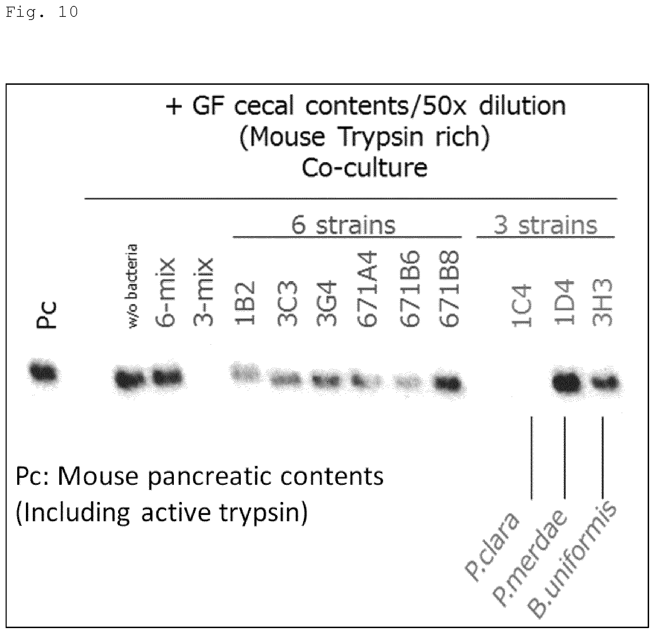

[Fig. 10] Fig. 10 is a photograph showing the results of evaluating the degradation of trypsin protein by isolated cultured bacteria. The cecal contents of GF mice containing trypsin and each bacterium were mixed and anaerobically cultured, and then the presence or absence of trypsin protein was evaluated by Western blotting. In the figure, "Pc" shows the results of evaluating the pancreatic contents of mice (including active trypsin), and "w/o bacteria" shows the results of evaluating the cecal contents of GF mice (unmixed with bacteria). The "6-mix" and "3-mix" show the results of evaluating the cecal contents of the mice mixed with the 6 bacterial strains and the 3 bacterial strains shown in Figs. 8 and 9. The "6 strains" shows the results of evaluating the cecal contents of the mice mixed with the above 6 bacterial strains (1B2 to 671B8). The "3 strains" shows the results of evaluating the cecal contents of the mice mixed with the above 3 bacterial strains (1C4 to 3H3). Loss of trypsin protein was observed only in P. clara monobacterium, which is one of the constituent bacteria of 3mix and 3mix. That is, it is considered that the trypsin activity is suppressed by the degradation of trypsin by the bacteria P. clara.

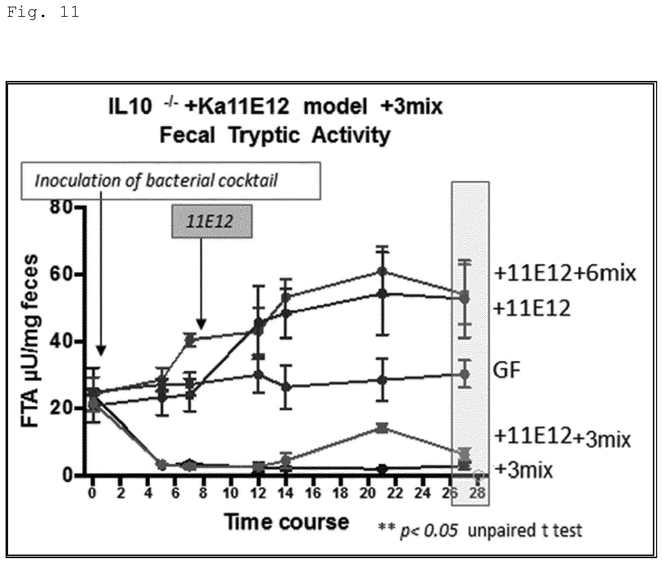

[Fig. 11] Fig. 11 is a graph showing the results of evaluating the suppression of the occurrence of ulcerative colitis (UC)-like inflammation due to the colonization of a trypsin activity-suppressing bacterial cocktail. IL10-/- mice were inoculated with the above-mentioned trypsin activity-suppressing bacteria 3mix or trypsin activity non-suppressing bacteria 6mix, and then infected with Ka11E12, which induces UC-like enteritis, and the mouse fecal trypsin activity was detected over time. In the figure, the horizontal axis shows the elapsed time since the inoculation of each bacterial cocktail. The vertical axis shows the fecal trypsin activity. The "11E12" shows the result of inoculation of only Ka11E12 to an IL10-/- mouse, and "11E12+6mix" and "11E12+3mix" show the results of inoculation of Ka11E12 to IL10-/- mice after inoculation of 3mix and 6mix. The "GF" shows the result of evaluating a germ-free mouse. The "3mix" shows the result of inoculation of only 3mix to an IL10-/- mouse.

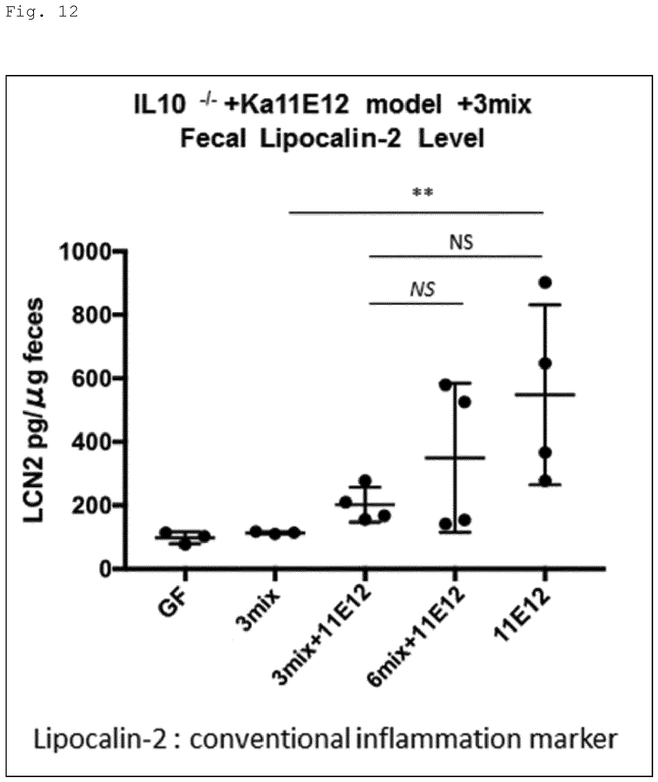

[Fig. 12] Fig. 12 is a dot plot diagram showing the results of evaluating the intestinal inflammation level 3 weeks after inoculation of Ka11E12 in the mice shown in Fig. 11. In the figure, the notation on the horizontal axis is the same as that in Fig. 11. The vertical axis shows the concentration of fecal inflammation markers (lipocalin-2, LCN2).

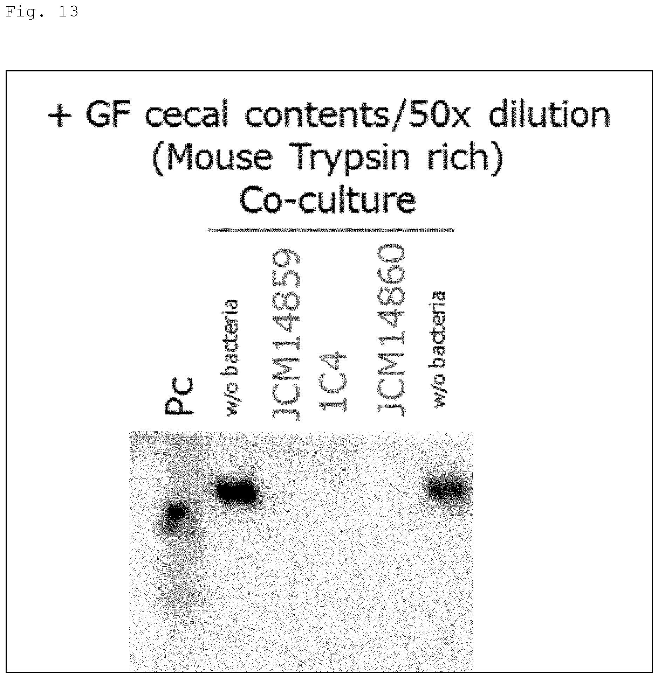

[Fig. 13] Fig. 13 is a photograph showing the results of evaluating the degradation of trypsin protein by P. clara and its related bacterial species. In the figure, "Pc" shows the results of evaluating the pancreatic contents of mice (including active trypsin), and "w/o bacteria" shows the results of evaluating the cecal contents of GF mice (unmixed with bacteria). The "1C4" shows the result of evaluating the cecal contents of a mouse mixed with the P. clara bacterial strain 1C4 (see Fig. 10) isolated in the present invention. The "JCM 14859" shows the result of evaluating the cecal contents of a mouse mixed with another strain of the bacteria P. clara different from 1C4. The "JCM 14860" shows the result of evaluating the cecal contents of a mouse mixed with the P. xylaniphila bacterial strain, which is a related species of the bacteria P. clara. As shown in Fig. 13, trypsin-degrading activity was observed in all the strains. Only the above-mentioned 2 species, JCM 14859^{T} and JCM 14860^{T}, are known as the bacteria belonging to Paraprevotella. From this, the trypsin-degrading activity is presumed to be an activity common to the genus Paraprevotella.

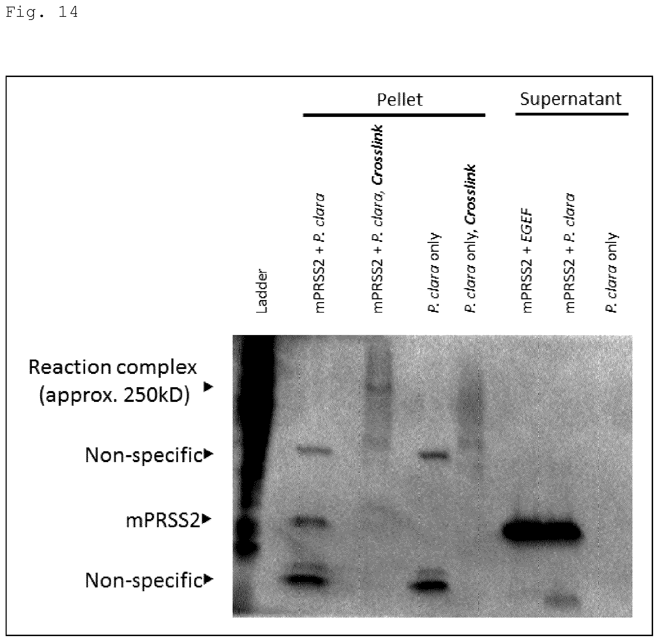

[Fig. 14] Fig. 14 is a photograph showing the results of a crosslink test between trypsin (mPRSS2) and a protein derived from the bacteria P. clara, analyzed by Western blotting using trypsin as a detection target. In the figure, "mPRSS2+P. clara" shows the result of analyzing the cell lysate of the bacteria P. clara simply incubated with mPRSS2, "mPRSS2+P. clara Crosslink" shows the result of analyzing the cell lysate of the bacteria P. clara incubated with mPRSS2 and further subjected to a crosslink reaction, "P. clara" shows the result of analyzing the cell lysate of P. clara without incubation with mPRSS2, and "P. clara Crosslink" shows the result of analyzing the cell lysate of P. clara not incubated with mPRSS2 but subjected to a crosslink reaction. The "mPRSS2+EGEF" shows the result of analyzing EGEF medium supplemented with mPRSS2, "mPRSS2+P. clara" shows the result of analyzing EGEF medium after incubating mPRSS2 and P. clara, and "P. clara only" shows the result of analyzing EGEF medium after incubating only P. clara. In the figure, "mPRSS2" shows the position of the band of mPRSS2 detected by Western blotting, and "Reaction complex (approx. 250 kDa)" shows the position of the band of the complex with the bacteria P. clara-derived protein (about 220 kDa) formed by adsorbing or binding to mPRSS2 (about 32 kDa). "Non-specific" shows the position of the non-specific band detected by Western blotting.

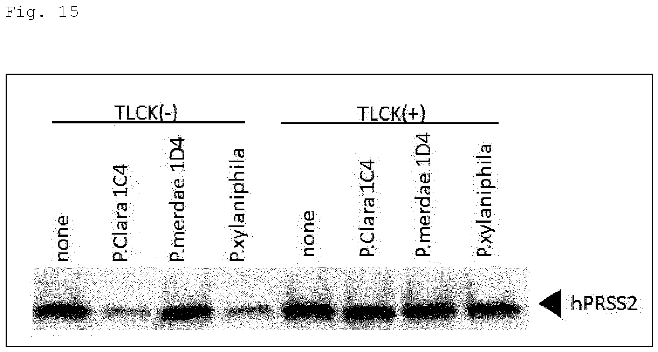

[Fig. 15] Fig. 15 is a photograph showing the results of a trypsin activity inhibition test. More specifically, it is a photograph showing the results of detecting hPRSS2 by Western blotting after incubating human PRSS2 (hPRSS2) treated with a trypsin activity inhibitor ("TLCK(+)" in the figure) or untreated hPRSS2 ("TLCK(-)" in the figure) and each of the bacteria (P. Clara 1C4, P. merdae 1D4, or P. xylaniphila). Note that in the figure, "none" shows the result of incubating only trypsin in the absence of bacteria.

### [Description of Embodiments]

As shown in Examples described later, it has been revealed that a bacterium belonging to a genus Paraprevotella suppresses the activity of trypsin. Therefore, the present invention provides a composition for suppressing trypsin activity, which contains a bacterium belonging to a genus Paraprevotella as an active ingredient.

"Trypsin" is a type of endopeptidase and serine protease. In addition, it is a type of digestive enzyme contained in pancreatic juice, and has an activity of hydrolyzing the peptide bond on the carboxy group side of a basic amino acid. The trypsin whose activity is suppressed in the present invention is not particularly limited, but is preferably anionic trypsin, and more preferably anionic-trypsin-2 (PRSS2). Typically, as for PRSS2, human-derived ones include a polypeptide composed of an amino acid sequence defined by NCBI reference sequence: NP_002761 (a polypeptide composed of an amino acid sequence encoded by a base acid sequence defined by NCBI reference sequence: NM_002770), and mouse-derived ones include a polypeptide composed of an amino acid sequence defined by NCBI reference sequence: NP_033456 (a polypeptide composed of an amino acid sequence encoded by a base acid sequence defined by NCBI reference sequence: NM_009430). Note that the PRSS2 according to the present invention is not limited to the polypeptides specified by these typical amino acid sequences, and also includes functionally active derivatives thereof, functionally active fragments thereof, homologues thereof, variants encoded by nucleic acids that hybridize to nucleic acids encoding these polypeptides under high stringency or low stringency conditions. In addition, such derivatives, fragments, homologues, or variants include polypeptides having at least 60% (preferably 70%, more preferably 80%, further preferably 90%, more preferably 95%, and particularly preferably 99%) homology to the particular amino acid sequences.

As described above, the "trypsin activity" usually means an activity of hydrolyzing a peptide bond on the carboxy group side of a basic amino acid (lysine, arginine). However, as shown in Examples described later, in the present invention, the suppression of trypsin activity is achieved by degradation of trypsin. It is also suggested in Examples described later that the degradation is induced by promoting the autolysis (autodigestion) of trypsin. Therefore, in the "trypsin activity" suppressed in the present invention, the activity of autolysis is excluded. In addition, in the present invention, the "suppression of trypsin activity" can be paraphrased as promotion of trypsin degradation or promotion of trypsin autolysis.

For example, the evaluation of the "suppression of trypsin activity" is possible by those skilled in the art by detecting the degree of degradation of a labeled substrate peptide using the label (for example, "Protease Activity Assay Kit (Abcam ab111750)" shown in Examples described later). In addition, it can also be done by assessing the degree of trypsin degradation by an immunological detection method (for example, Western blotting) that uses trypsin as the detection target, as shown in the Examples described later. Note that in the present invention, "suppression" also includes complete suppression (inhibition).

In the composition of the present invention, the "bacterium belonging to the genus Paraprevotella" contained as an active ingredient for suppressing trypsin activity includes bacteria belonging to the phylum Bacteroides, the class Bacteroidia, the order Bacteroidales, the family Prevotellaceae, and the genus Paraprevotella, and examples thereof include Paraprevotella clara (P. clara) and Paraprevotella xylaniphila (P. xylaniphila).

Examples of P. clara include a bacterial strain belonging to Paraprevotella clara specified by accession number NITE BP-02775 (Paraprevotella clara 1C4 strain), and a Paraprevotella clara JCM 14859^{T} strain.

Examples of P. xylaniphila include Paraprevotella xylaniphila JCM 14860^{T} strain.

Note that the "Paraprevotella clara JCM 14859^{T} strain" and the "Paraprevotella xylaniphila JCM 14860^{T} strain" can be obtained from Japan Collection of Microorganisms (JCM), RIKEN BioResource Research Center (RIKEN BRC).

In addition, an example of the bacterium belonging to the genus Paraprevotella is at least one bacterium having a DNA composed of a base sequence set forth in any of SEQ ID NOs: 1 to 3 or a base sequence having at least 90% identity to the base sequence. Note that the base sequences set forth in SEQ ID NOs: 1 to 3 indicate the 16S rRNA sequences of the Paraprevotella clara 1C4 strain, the Paraprevotella clara JCM 14859^{T} strain, and the Paraprevotella xylaniphila JCM 14860^{T} strain, respectively.

In addition to the above-mentioned bacterium belonging to the genus Paraprevotella, from the viewpoint of facilitating colonization of the bacterium in the intestine, the composition for suppressing trypsin activity of the present invention may also further contain at least one bacterium selected from the group consisting of Parabacteroides merdae and Bacteroides uniformis

Examples of "Parabacteroides merdae" include a bacterial strain belonging to Parabacteroides merdae specified by accession number NITE BP-02776 (Parabacteroides merdae 1D4 strain). In addition, an example of Parabacteroides merdae is at least one bacterium having a DNA composed of a base sequence set forth in SEQ ID NO: 4 or a base sequence having at least 90% identity to the base sequence. Note that the base sequence set forth in SEQ ID NO: 4 indicates the sequence of 16S rRNA of the Parabacteroides merdae 1D4 strain.

Examples of Bacteroides uniformis include a bacterial strain belonging to Bacteroides uniformis specified by accession number NITE BP-02777 (Bacteroides uniformis 3H3 strain). In addition, an example of Bacteroides uniformis is at least one bacterium having a DNA composed of a base sequence set forth in SEQ ID NO: 5 or a base sequence having at least 90% identity to the base sequence. The base sequence set forth in SEQ ID NO: 5 indicates the sequence of 16S rRNA of the Bacteroides uniformis 3H3 strain.

Note that the "at least 90% identity" in the present invention means that the identity to each base sequence is preferably 95% or more (for example, 96% or more, 97% or more, and 98% or more), and particularly preferably 99% or more.

The bacteria contained in the composition of the present invention may be live bacteria or dead bacteria. In addition, they may be substances contained in the bacteria (such as proteins, nucleic acids, lipids, sugars, and sugar chains), secretory products of the bacteria, or metabolites of the bacteria. In addition, the compositions of the present invention can be used in combination, and when ingested or absorbed in combination as a result (in the case of a combination composition), the above-mentioned bacteria can also be present separately in 2 or more compositions.

The composition for suppressing trypsin activity of the present invention can be in the form of a pharmaceutical composition (such as pharmaceuticals and quasi-drugs), food and drink (including animal feed), or a reagent used for research purposes (such as in-vitro or in-vivo experiments). In addition, since the composition of the present invention suppresses trypsin activity, it is used as a pharmaceutical composition or food and drink for treating, preventing, or ameliorating a disease caused by the activity.

The composition of the present invention can be formulated by a known pharmaceutical method. For example, capsules, tablets, pills, liquids, powders, granules, fine granules, film coating agents, pellets, troches, sublingual preparations, chews, buccal agents, pastes, syrups, suspensions, elixirs, emulsions, coatings, ointments, plasters, poultices, transdermal formulations, lotions, inhalants, aerosols, injections, suppositories, and the like can be used for inoculation by oral, parenteral (for example, intestinal, intramuscular, intravenous, intratracheal, intranasal, transdermal, intradermal, subcutaneous, intraocular, vaginal, intraperitoneal, rectal, or inhalational), or a combination of these routes.

These formulations can be appropriately combined with a pharmacologically or food and drink-acceptable carrier such as, specifically, sterilized water, physiological saline, buffer solution, medium, vegetable oil, solvent, base, emulsifier, suspension, surfactant, stabilizer, flavoring agent, air freshener, excipient, vehicle, preservative, binder, diluent, isotonic agent, soothing agent, bulking agent, disintegrant, buffer agent, coating agent, lubricant, colorant, sweetener, thickener, flavor modifier, solubilizer, or other additives.

Further, among these formulations, from the viewpoint of more efficiently suppressing trypsin activity in the intestinal tract, formulations intended for oral inoculation may be particularly combined with a composition that enables efficient delivery of the composition of the present invention into the intestinal tract. The compositions capable of such delivery into the intestinal tract are not particularly limited, and known compositions can be appropriately employed. Examples thereof include pH-sensitive compositions, compositions that suppress release to the intestinal tract (such as cellulose-based polymers, acrylic acid polymers and copolymers, and vinyl acid polymers and copolymers), bioadhesive compositions that specifically adhere to the intestinal mucosa (such as the polymer described in U.S. Patent No. 6,368,586), compositions containing protease inhibitors, and compositions that are specifically degraded by intestinal enzymes.

In addition, when used as a pharmaceutical composition, it may further contain a known substance used for treating, preventing, or ameliorating a disease caused by trypsin activity (such as antiinflammatory agents and immunosuppressants), or may be used in combination with such a substance.

When the composition of the present invention is used as food and drink, the food and drink may be, for example, a health food, a functional food, a food for specified health use, a food with nutritional functions, a food with functional claims, a nutritional supplement, a food for the sick, or animal feed. Specific examples of the food and drink include liquid foods such as fermented beverages, oil-containing products, soups, milk beverages, soft drinks, tea beverages, alcoholic beverages, energy drinks, and jelly-like beverages, carbohydrate-containing foods, processed livestock foods, and processed marine products; processed vegetable foods, semi-solid foods, fermented foods, confectionery, retort pouch products, and microwave oven-compatible foods. Examples also further include health foods and drinks prepared in the form of powder, granules, tablets, capsules, liquid, paste, or jelly. Note that the food and drink according to the present invention can be produced by a production technique known in the art. The food and drink may be added with ingredients (such as nutrients) effective for improving or preventing diseases caused by trypsin activity. In addition, it may be multifunctional food and drink by combining it with other ingredients or other functional foods that exhibit functions other than the improvement.

The product of the composition of the present invention (such as a pharmaceutical product, a quasi-drug, food and drink, or a reagent) or an instruction manual thereof may be labeled to be used for suppressing trypsin activity or for treating, ameliorating, or preventing diseases caused by trypsin activity. In addition, regarding the food and drink, so that the form, the target, and the like can be distinguished from those of general foods, the label of health functions as a health functional food (food for specified health use, food with nutritional functions, or food with functional claims) may be attached to the product of the composition of the present invention. Here, "product or instruction manual is labeled" means that a label is attached to the main body, container, package, or the like of the product, or that a label is attached to an instruction manual, package insert, promotional material, other printed matter, or the like that discloses product information. Further, the composition of the present invention may be in the form of a kit.

In addition, as described above, a pharmaceutical composition can be produced by a known formulation technique using the above-mentioned bacteria and the like. Therefore, the present invention also provides the use of the intestinal bacteria and the like of the present invention for producing a pharmaceutical composition for treating, ameliorating, or preventing a disease caused by trypsin activity.

### <Treatment Method and the Like for Diseases Caused by Trypsin Activity>

The present invention also provides a method for suppressing trypsin activity in a target, a method for suppressing immunity in the target, or a method for treating, ameliorating, or preventing a disease caused by trypsin activity in the target, including allowing the target to ingest the above-mentioned composition or the above-mentioned bacteria serving as an active ingredient thereof (also referred to as the "pharmaceutical compositions of the present invention or active ingredients thereof").

In the present invention, the "disease caused by trypsin activity" means a disease induced by trypsin activity, and examples thereof include inflammatory bowel disease (chronic inflammatory bowel disease such as ulcerative colitis, Crohn's disease, and inflammatory bowel disease).

In the present invention, "treating, improving" includes not only complete recovery from the disease, but also alleviation of the symptoms of the disease or suppression of its progression. The "preventing" includes suppressing or delaying the onset of the disease and suppressing its recurrence.

The pharmaceutical compositions of the present invention or active ingredients thereof can be used for animals including humans as the target, but there are no particular restrictions on animals other than humans, and various domestic animals, poultry, pets, laboratory animals, and the like can be used as the target. In addition, the ingestion target of the pharmaceutical compositions of the present invention or active ingredients thereof include not only those suffering from a disease caused by trypsin activity, but also those suspected to be suffering from the disease and those in which the symptoms of the disease are alleviated or eliminated (relieved).

The method of ingesting the pharmaceutical compositions of the present invention or active ingredients thereof is not particularly limited, and may be oral inoculation or parenteral inoculation (for example, inoculation into the intestinal tract). In the case of oral inoculation, from the viewpoint of further improving the effects of the pharmaceutical compositions of the present invention or active ingredients thereof, it is preferable that the ingestion target of the pharmaceutical compositions of the present invention or active ingredients thereof reduces the production of gastric acid by ingestion of a proton-pump inhibitor (PPI) or the like.

In addition, when the pharmaceutical compositions of the present invention or active ingredients thereof are ingested, the amount ingested can be appropriately selected by those skilled in the art according to the age, weight, symptoms of the disease, health conditions, type of the composition (such as a pharmaceutical and food and drink), ingestion method, and the like.

Preferable embodiments of the composition or method for treating, ameliorating, or preventing a disease caused by trypsin activity of the present invention have been described above, but the composition or method is not limited to the above embodiments.

As shown in Examples described later, inoculation of a bacterium belonging to a genus Paraprevotella, Parabacteroides merdae, and Bacteroides uniformis tended to suppress the onset in a model in which IL-10 gene-deficient mice are infected with Enterobacter aerogenes to induce colitis. In addition, the onset of colitis was significantly suppressed also in the DSS-induced colitis model.

Therefore, the present invention also provides pharmaceutical compositions and methods for treating, ameliorating, or preventing the following diseases caused by trypsin activity.

A pharmaceutical composition for treating, ameliorating, or preventing a disease caused by trypsin activity, comprising: at least one bacterium selected from the group consisting of a bacterium belonging to a genus Paraprevotella, Parabacteroides merdae, and Bacteroides uniformis as an active ingredient.

A method for treating, ameliorating, or preventing a disease caused by trypsin activity in a target by allowing the target to ingest at least one bacterium selected from the group consisting of a bacterium belonging to a genus Paraprevotella, Parabacteroides merdae, and Bacteroides uniformis.

### <Novel Bacteria>

As shown in Examples described later, the following 3 bacterial strains are Staphylococcus aureus bacterial strains isolated and cultured for the first time by the present inventors. In addition, the usefulness thereof is also as described above. Therefore, the present invention also provides the following bacterial strains.

A bacterial strain belonging to Paraprevotella clara specified by accession number NITE BP-02775 (Paraprevotella clara 1C4 strain).

A bacterial strain belonging to Parabacteroides merdae specified by accession number NITE BP-02776 (Parabacteroides merdae 1D4 strain).

A bacterial strain belonging to Bacteroides uniformis specified by accession number NITE BP-02777 (Bacteroides uniformis 3H3 strain).

Note that all of the bacterial strains were deposited at Patent Microorganisms Depositary, National Institute of Technology and Evaluation (NITE) (postal code 292-0818 2-chome-5-8 Kazusakamatari, Kisarazu, Chiba, room number 122) on August 30, 2018.

### [Examples]

Hereinafter, the present invention is described in more detail based on Examples, but the present invention is not limited to the following Examples. In addition, the present Example was carried out using the materials and methods shown below.

### (Germ-Free Mouse)

C57BL/6N Jcl gnotobiotic mice (CLEA Japan, Inc., 4 to 8 weeks of age) were bred in a breeding vinyl isolator (germ-free isolator, ICM Co., Ltd.; ICM-1B) for 1 week or more under the conditions of free water intake and feeding to acclimatize to the environment.

### (SPF Mouse)

C57BL/6N SPF mice (4 to 8 weeks of age) were obtained from RIKEN BioResource Research Center, Japan SLC, Inc., CLEA Japan, Inc., or Charles River Laboratories Japan, Inc., and bred in an SPF environment under the conditions of free water intake and feeding for 1 week or more to acclimatize to the environment.

### (Proteome Analysis of Cecal Contents)

The mice were sacrificed under isoflurane anesthesia, and the cecal contents were harvested and stored at -80°C. The cryopreserved sample was thawed on ice, and a 5-time weight of RIPA Lysis Buffer with protease inhibitor cocktail (Cosmo Bio; AKR-190) was added and sufficiently stirred. The mixture was centrifuged at 15,000 × g for 20 minutes at 4°C to obtain a supernatant with proteins dissolved therein.

A 30% trichloroacetic acid (TCA) solution (Nacalai Tesque, Inc.; 37211-55) in the same volume as the protein solution was mixed, which was allowed to stand at 4°C for 30 minutes and then centrifuged at 15,000 × g for 20 minutes at 4°C to remove the supernatant, thereby obtaining a protein precipitate. The precipitate was purified by repeating redispersion and reprecipitation in acetone twice.

A 100 mM Tris-HCl pH 9.0 buffer solution (NIPPON GENE CO., LTD.; 316-90385) dissolved with final concentrations of 12 mM sodium deoxycholate (SDC, Nacalai Tesque, Inc.; 02889-72) and 12 mM sodium lauryl sulfate (SLS, Nacalai Tesque, Inc.; 31623-32) was added so that the protein concentration was 2.0 µg/µL, and sonication for 5 seconds was repeated 4 times for redissolution. TaKaRa BCA Protein Assay Kit (Takara Bio Inc.; T9300A) was used to quantify by the BCA method the concentration of protein, which was diluted to 1.0 µg/µL in the measuring cylinder.

To 20 µL of a 1.0 µg/µL protein solution, 2 µL of an aqueous solution of 100 mM dithiothreitol (Nacalai Tesque, Inc.; 14128-91) was added, and the mixture was heated at 50°C for 30 minutes to reduce disulfide bonds. Further, 2 µL of an aqueous solution of 375 mM iodoacetamide (Nacalai Tesque, Inc.; 19302-54) was added and reacted at room temperature for 30 minutes to alkylate the thiol groups. Then, 4 µL of an aqueous solution of 400 mM cysteine (Nacalai Tesque, Inc.; 11548-52) was added and reacted for 10 minutes to quench the remaining iodoacetamide. After that, 80 µL of an aqueous solution of 50 mM ammonium hydrogen carbonate (Nacalai Tesque, Inc.; 08887-54), 2 µL of a solution of 200 ng/µL lysyl endopeptidase (Wako Pure Chemical Industries, Ltd.; 125-05061), and 2 µL of a solution of 200 ng/µL trypsin (Wako Pure Chemical Industries, Ltd.; 202-15951) were added and reacted at 37°C overnight to form the proteins into peptide fragments.

After adding 30 µL of an aqueous solution of 5% trifluoroacetic acid (TFA, Nacalai Tesque, Inc.; 34901-21), liquid-liquid extraction was performed with 200 µL of ethyl acetate (Nacalai Tesque, Inc.; 14747-65) to remove SDC and SLS. The dissolved organic solvents were removed by centrifugal evaporator treatment. Then, 100 µL of an aqueous solution of 0.1% TFA was added, and the mixture was centrifuged at room temperature and 15,000 × g for 15 minutes to remove insoluble matter. After desalting with C18 Tips (Thermo Fisher Scientific; 87782), the mixture was dissolved in an aqueous solution with final concentrations of 3% acetonitrile (Nacalai Tesque, Inc.; 00430-25) and 0.1% formic acid (Nacalai Tesque, Inc.; 08965-82). LC-MS measurement was performed using the SCIEX TripleTOF 5600 System, and proteome analysis was performed using SWATH Acquisition.

### (Obtaining Mouse Feces or Human Healthy Volunteer Feces)

The feces of healthy volunteers #A to G or mouse fecal samples were diluted 5 times by weight with 20% by volume of glycerol-dissolved PBS, filtered through a 100 µm diameter filter, and stored as a stock solution at -80°C.

### (Measurement of Fecal Trypsin Activity)

The frozen fecal sample were thawed at room temperature, dispersed in a 0.9% NaCl aqueous solution, and the trypsin activity of the supernatant was measured according to the protocol of Protease Activity Assay Kit (Abcam ab111750).

### (Preparation of Mice Colonized with Bacteria Derived from Human Healthy Volunteer Feces)

The stock solution prepared above (Obtaining Mouse Feces or Human Healthy Volunteer Feces) was melted at room temperature and diluted with PBS to a 10-fold volume. Then, 200 µL of the diluted solution was orally inoculated into the stomach of germ-free mice. For another month, the mice were bred in a germ-free isolator under the conditions of free water intake and feeding to colonize the mice with the bacteria in the transplanted feces. In addition, the trypsin activity of these mice was also measured by the method described in the above (Measurement of Fecal Trypsin Activity).

### (Preparation of Mice Colonized with Bacteria Derived from Human Healthy Volunteer Feces and Elimination of Colonized Bacteria by Antibiotic Inoculation)

In the above (Preparation of Mice Colonized with Bacteria Derived from Human Healthy Volunteer Feces), free drinking water was changed to a 200 mg/L aqueous solution of ampicillin, metronidazole, or tylosin, and the animals were bred for another month to eliminate bacteria non-resistant to each antibiotic. The fecal samples of the mice during the inoculation process were obtained by the method described above (Obtaining Mouse Feces or Human Healthy Volunteer Feces), and trypsin activity was measured by the method described above (Measurement of Fecal Trypsin Activity).

### (DNA Extraction from Mouse Feces)

To 100 µl of fecal sample solution filtered with a 100 µm diameter filter, 800 µl of 10 mM Tris/10 mM EDTA buffer (pH 8.0, hereinafter referred to as TE10) dissolved with 15 mg lysozyme (Sigma-Aldrich, Lysozyme from chicken egg white; L4919) and 5 µl RNase (Thermo Fisher Scientific, PureLink RNase A (20 mg/mL); 12091-021) was added, and the mixture was shaken at 37°C for 1 hour. Subsequently, 2,000 U of Achromopeptidase (registered trademark) (Wako; 015-09951) was added, and the mixture was shaken at 37°C for 30 minutes for lysis.

Then, 50 µl of 20% SDS TE10 solution and 50 µl of TE10 solution dissolved with 20 mg/ml proteinase K (Roche, Proteinase K, recombinant, PCR Grade; 03115852001) were added, and the mixture was shaken at 55°C for 60 minutes.

DNA was extracted by a liquid-liquid extraction method using Phenol/Chloroform/Isoamyl alcohol (25:24:1) (Wako; 311-90151), and bacterial genomic DNA was obtained by ethanol precipitation.

### (Analysis of Microbiota in Mouse Feces)

A PCR reaction was performed on the bacterial genomic DNA using TaKaRa ExTaq (Takara Bio Inc.; RR001A) to prepare Illumina Miseq sequence amplicons for 16S rDNA. The primer sequence is as follows (Nishijima S et al DNA Res 23 125-133 2016). 27Forward-mod: (5'-AATGATACGGCGACCACCGAGATCTACAC**index**ACACTCTTTCCCTACA CGACGCTCTTCCGATCTAGRGTTTGATYMTGGCTCAG-3')

Both sequences adjacent to the index sequence are set forth in SEQ ID NOs: 10 and 11. 338Reverse: (5'-CAAGCAGAAGACGGCATACGAGAT**index**GTGACTGGAGTTCAGACGTGT GCTCTTCCGATCTTGCTGCCTCCCGTAGGAGT-3')

Both sequences adjacent to the index sequence are set forth in SEQ ID NOs: 12 and 13.

The amplicons were purified using Agencourt AMPure (registered trademark) XP (Beckman Coulter; A63882) and Min Elute PCR Purification Kit (Qiagen; 28004), and the DNA concentration of the amplicons was quantified using Quant-iT PicoGreen (registered trademark) dsDNA Assay Kit (Thermo Fisher Scientific; P7589) and KAPA library Quantification kit (Roche Diagnostics; 07960166001). The average DNA length of the mixture of amplicons was measured using the Agilent 2100 Bioanalyzer High Sensitivity DNA Kit (Agilent Technologies; 5067-4626), and the molar concentration of the amplicons was calculated. The library was denatured and a hybridization solution was prepared according to the Illumina Miseq 16S rDNA genome analysis protocol, and sequencing was performed using Miseq (Illumina; SY-410-1003) and Miseq Reagent Kit v3 (Illumina; MS-102-3003).

From the obtained sequence data, 3000 reads with high quality were randomly selected for each sample, and microbiota data was obtained by bacterial species assignment by the database assignment method for the representative sequences of operational taxonomic unit (OTU). For the database, NCBI (https://www.ncbi.nlm.nih.gov/taxonomy) and RDP (http://rdp.cme.msu.edu/) were used, and among the top hits of GLSEARCH (http://nebc.nox.ac.uk/bioinformatics/docs/glsearch.ht ml) for each database, the most homologous results were employed for strain assignment (Nishijima S et al DNA Res 23 125-133 2016).

### (Isolation of Bacteria)

The feces 12 days after inoculation of fecal samples were diluted with PBS and cultured in a 10% CO₂ anaerobic environment using the following media, and the colonies formed were isolated.

With EG ext. medium, mGAM medium, Schaedler medium (Wako; 517-45805), BL medium (Nissui;5430), and CM0619 medium (Wilkins T. D. and Chalgren S. (1976) Antimicrob. Agents Chemother. 10. 926-928.) as the basal media, an agar plate of a medium supplemented with horse blood or defibrinated horse blood having a final concentration of 5% was used. In particular, as for CM0619 medium, SR0107 selective medium or SR0108 selective medium supplemented with a predetermined supplement (http://www.oxoid.com/UK/blue/prod_detail/prod_detail. asp?pr=CM0619) was also used in the same manner.

### (Creation of Phylogenetic Tree)

The package ape of the statistical analysis software R (https://www.r-project.org/) was used to create a phylogenetic tree based on the similarity of the 16S rDNA sequences of the isolated 35 bacteria based on the representative sequences of OTU used for assignment at the time of microbiota analysis.

### (Suppression of Intestinal Trypsin Activity by Colonization of 35 Isolated Bacteria)

Each bacterium isolated in the above (Isolation of Bacteria) was cultured in EG ext. medium, mGAM medium, Schaedler medium, BL medium, or CM0619 medium in a 10% CO₂ anaerobic environment at 37°C for 1 to 3 days. Equal volumes of the bacterial solutions that had reached the steady state were mixed, and 200 µL thereof was orally inoculated into the stomach of the germ-free mice of Example 1. The bacteria were colonized by breeding in a germ-free isolator under the conditions of free water intake and feeding. In addition, trypsin activity was measured according to the above (Measurement of Fecal Trypsin Activity).

### (Spearman's Rank Correlation Analysis)

Spearman's rank correlation analysis was performed on the relative occupancy of each constituent bacterium and the fecal trypsin activity value thereof obtained from the intestinal microbiota analysis based on 16S rDNA 12 days after the fecal inoculation. The bacterial group in which the correlation coefficient ρ between the relative occupancy of the bacteria and the trypsin activity value was ρ ≤ -0.5 and the P-value of the uncorrelated test was P < 0.05 was estimated as a candidate for trypsin activity-suppressing bacteria.

### (Suppression of Intestinal Trypsin Activity by Selective Bacterial Cocktail)

In the above (Spearman's Rank Correlation Analysis), regarding the 9 bacterial strains estimated as candidates for trypsin activity-suppressing bacteria, bacterium-colonized mice were prepared and their intestinal trypsin activity was measured according to the method described above (Suppression of Intestinal Trypsin Activity by Colonization of 35 Isolated Bacteria). In addition, the 9 bacterial strains were divided into 3 Bacteroides bacterial strains and 6 non-Bacteroides bacterial strains, and bacterium-colonized mice were prepared and their intestinal trypsin activity was measured.

### (Suppression of Ulcerative Colitis (UC)-Like Inflammation by Colonization 6 of Trypsin Activity-Suppressing Bacterial Cocktail)

IL10-/- mice (4 to 8 weeks of age, The Jackson Laboratory) were aseptically bred according to the method described above (germ-free mice). In addition, 3 isolated Bacteroides bacterial strains or 6 non-Bacteroides bacterial strains were orally inoculated and colonized according to the method described above (Suppression of Intestinal Trypsin Activity by Selective Bacterial Cocktail). One week after the oral inoculation, UC-like inflammation-inducing treatment was performed according to the following procedure. The K. aeromobilis 11E12 strains (Atarashi K. et al Science 358, 359 2017) isolated from UC patients were cultured in Schaedler medium at 37°C in a 10% CO₂ anaerobic environment for 1 to 3 days, and 1 to 2 × 10⁸ CFU/200 µl of the bacterial solution that had reached the steady state was orally inoculated into the stomach of the mice. Subsequently, they were bred in a germ-free isolator under the conditions of free water intake and feeding for another 3 weeks to colonize the bacteria. The intestinal trypsin activity was measured 3 weeks after the inoculation of the 11E12 strains according to the method described above (Measurement of Fecal Trypsin Activity). In addition, as an index of inflammation, the fecal level of Lipocalin-2 was measured by the ELISA method (abcam; Mouse Lipocalin-2 ELISA Kit; ab199083).

### (Suppression of Trypsin Activity by Cultured Bacteria)

To the culture solution of each of the 9 bacteria selected in the above (Spearman's Rank Correlation Analysis) that had reached the steady state after culturing in a 10% CO₂ anaerobic environment at 37°C for 1 to 3 days, a trypsin-containing solution obtained by diluting the cecal contents of germ-free mice 50-fold with germ-free water was mixed in the same volume, which was further cultured under the same conditions for 12 hours.

Centrifugation was carried out at 10,000 × g for 15 minutes to obtain a supernatant free of bacterial cells. They were mixed with a loading buffer (BioRad; 1610739), and after mercaptoethanol reduction and heat denaturation treatment, SDS-tricine PAGE was performed. Subsequently, a voltage was applied in Tris-glycine buffer and transferred to Immobilon-P Transfer Membrane (Merck; ISEQ07850). The anti-anionic trypsin-2 rabbit antibody was used as the primary antibody, and Anti-IgG (H + L chain) (Rabbit) pAb-HRP (MBL; Code No. 458) was used as the secondary antibody, and the presence or absence of the anionic trypsin-2 protein was confirmed using chemiluminescence by Chemi-Lumi One Super (Nacalai Tesque, Inc.; 02230-14).

### (Suppression of Trypsin Activity by Paraprevotella Bacteria)

JCM 14859^{T} (P. clara strain) and JCM 14860^{T} (P. xylaniphila strain) were obtained from Japan Collection of Microorganisms (JCM), RIKEN BioResource Research Center (RIKEN BRC), and were each cultured in EG ext. medium under 37°C and 10% CO₂ anaerobic environment for 1 to 3 days. The bacterial solution that had reached the steady state was treated according to the method described above (Suppression of Trypsin Activity by Cultured Bacteria), and the degrading activity of trypsin was evaluated from the results of Western blotting.

### (Crosslink Test between P. Clara-Derived Protein and Trypsin)

First, 25 µl of 150 µg/ml mPRSS2/PBS solution, 150 µl of P. clara 1C4 strain (OD600 > 1.0) that had been cultivated in EGEF medium under anaerobic conditions and had reached the steady phase, and 75 µl of fresh EGEF medium were mixed and incubated at 37 °C for 35 minutes. Centrifugation was carried out at room temperature at 4,000 × g for 5 minutes, and the supernatant was collected (corresponding to the "supernatant" sample in Fig. 14). After redispersing the bacteria in 800 µl of PBS, they were centrifuged at 4,000 × g at room temperature to remove the supernatant, thereby removing the unadsorbed mPRSS2 protein. The bacteria were redispersed in 250 µl of an aqueous solution of 10 mM DSSO/PBS and allowed to stand at room temperature for 15 minutes to form random covalent crosslinks in the bacterial constituents and adsorbents. Further, 30 µl of an aqueous solution of 200 mM Tris-HCl (pH 8.0) was added, and the mixture was allowed to stand at room temperature for 5 minutes to quench the unreacted DSSO. Centrifugation was carried out at under the conditions of 4,000 × g and room temperature for 5 minutes, and the supernatant was removed. After redispersing the bacteria in 800 ul of PBS, they were centrifuged under the same conditions, and the supernatant was removed and washed. The bacteria were disrupted by adding a cell lysate having a 1% SDS/10 mM Tris-HCl (pH 8.0)/5 mM EDTA composition, and then were centrifuged under the conditions of 20,000 × g at 4°C for 5 minutes to obtain a cell disruption solution as a supernatant (corresponding to the "Pellet" sample in Fig. 14). The supernatant sample and the Pellet sample were subjected to Western blotting, and the mPRSS2-specific antibody was detected by chemiluminescence to detect the protein bound to mPRSS2. Note that the reagents and the like used in the present method are as follows.
mPRSS2: mouse recombinant PRSS2 protein (His Tag) (Sino biological 50383-M08H)
DSSO: disuccinimidyl sulfoxide (Thermo Fisher Scientific A33545)
SDS: prepared from 10%-SDS solution (Nacalai Tesque, Inc. 30562-04)
Tris-HCl: prepared from 1 mol/l-Tris-hydrochloric acid buffer solution (pH 8.0) (Nacalai Tesque, Inc. 06938-44)
EDTA: prepared from 0.5 mol/l-EDTA solution (ph8.0) (Nacalai Tesque, Inc. 06894-14)
PBS: Dulbecco phosphate-buffered saline (free of Ca and Mg) (Nacalai Tesque, Inc. 14249-95)
Primary antibody for WB detection: Anti-6-His, Rabbit-Poly (Funakoshi Co., Ltd. A190-114A, used after diluting 400 times)
Secondary antibody for WB detection: Anti-IgG (Rabbit) pAb-HRP (MBL Code No. 458, used after diluting 400 times)
WB detection reagent: Chemi-Lumi One L (Nacalai Tesque, Inc. 07880-54).

### (Trypsin Activity Inhibition Test)

A 5-fold mol amount of TLCK DMSO solution was added to an aqueous solution of 1 mg/ml hPRSS2/PBS and allowed to stand at room temperature for 3 hours to obtain TLCK-modified TLCK-hPRSS2 having no trypsin activity. Unreacted TLCK was removed by Sephadex-G25 column chromatography, and TCLK-hPRSS2 was purified by concentration with an ultrafiltration membrane. The bacteria P. clara that had reached the steady state after culturing in EGEF medium under anaerobic conditions were centrifuged at room temperature at 4,000 × g for 5 minutes to redisperse the bacteria in PBS, and then, centrifugation and supernatant removal under the same conditions were performed once more to obtain bacteria from which the EGEF medium component had been removed. The bacteria dispersed in PBS and the TCLK-hPRSS2/PBS solution were mixed and allowed to stand for 3 hours in an anaerobic 37°C environment under the conditions of 20 ng/µl TLCK-hPRSS2 and the bacterial concentration OD = 0.9. The bacterial solution was centrifuged at room temperature at 4,000 × g for 5 minutes, and hPRSS2 in the supernatant was detected by Western blotting. The reagents and the like used in the present method are as follows.
hPRSS2: human PRSS2/trypsin 2 protein (recombinant) (LSBio LS-G20167-5)
TLCK: trypsin inhibitor tosyl-L-lysyl-chloromethane hydrochloride (ABCAM ab144542)
Sephadex-G25: Prepack Disposable PD-10 Column (GE Healthcare 17085101)
Primary antibody for WB detection: Anti-PRSS2/Trypsin 2 antibody IHC-plus (LSBio LS-B15185-50, used after diluting 400 times)
Secondary antibody for WB detection: Anti-IgG (Rabbit) pAb-HRP (MBL Code No. 458, used after diluting 400 times)
WB detection reagent: Chemi-Lumi One L (Nacalai Tesque, Inc. 07880-54).

### (Example 1)

### [Abundant Active Trypsin in Feces of Germ-Free Mice]

Comprehensive protein analysis (shotgun proteomics, proteome analysis) of the cecal contents of SPF mice (RIKEN·SPF mice) and GF mice maintained at RIKEN was performed.

As a result, although not shown in the figure, 713 host-derived proteins were detected, and among them, 45 proteins were significantly abundant in the cecal contents of the GF mice. Among these, attention was paid to the proteolytic enzyme trypsin (anionic trypsin-2: PRSS2), which is particularly abundant and has been reported to be associated with IBD.

Next, the activity of trypsin in the feces of RIKEN·SPF mice and GF mice was measured, and the amount of protein was evaluated based on Western blotting (WB).

As a result, although not shown in the figure, the trypsin activity was significantly higher in the GF mice than in the RIKEN·SPF mice. In addition, the amount of trypsin protein was higher in the GF mice. Furthermore, when the cross section of the intestinal tract of the distal large intestine of the RIKEN/SPF mice and GF mice was observed by immunohistochemical staining, a large amount of trypsin was present in the GF mice.

From the above, it has been found that more active trypsin is present in the large intestine beyond the cecum of the GF mice as compared with the RIKEN·SPF mice.

### [Abundant Active Trypsin in Feces of Inflammatory Bowel Disease and Inflammation Model Mice]

The activity of trypsin was measured and the amount of its protein was evaluated on the fecal samples of Japanese IBD patients suffering from ulcerative colitis (UC) and Crohn's disease (CD). As comparison targets, fecal samples of healthy Japanese volunteers were evaluated. As a result, although not shown in the figure, significantly more active trypsin was observed in the feces of UC and CD patients as compared with the feces of healthy individuals.

In addition, when the fecal trypsin activity during inflammation of IL-10 gene-deficient mice (IL10-/- mice) known as colitis model mice was evaluated, the trypsin activity was significantly higher than the fecal trypsin activity of WT mice. It has been reported that active trypsin remaining in the large intestine may be involved in the onset of IBD and the exacerbation of its pathological condition. The above series of results supports the existing reports.

### [Active Trypsin Activity Remains beyond Cecum in GF Mice]

The trypsin activity of each site in the digestive tract was compared between RIKEN·SPF mice and GF mice. As a result, although not shown in the figure, no significant difference was observed between the two in the small intestine, but it was significantly higher in the GF mouse beyond the cecum. In addition, when the expression level and secretion amount of the trypsin precursor trypsinogen (PRSS2) in the pancreatic tissues mainly responsible for the secretion of trypsin were confirmed by RT-qPCR and WB, no significant difference was observed between the two.

From the series of results, it is considered that the increase in trypsin activity in GF mice beyond the cecum is due to the abnormal residual of trypsin normally secreted from pancreatic tissues. One of the factors is considered to be the involvement of intestinal bacteria that colonize beyond the cecum. That is, it was assumed that in SPF mice, intestinal bacteria colonized beyond the cecum controlled the function of reducing active trypsin, and in GF mice, active trypsin remained due to the absence of intestinal bacteria.

### [Intestinal Microbiota beyond Cecum of Mice Affects Its Trypsin Activity]

It is known that the intestinal microbiota of SPF mice is uniquely maintained in each breeding facility. Therefore, in order to evaluate the relationship between the colonized intestinal bacteria beyond the cecum and its trypsin activity, the trypsin activity in the cecal contents and its microbiota were evaluated for SPF mice derived from different breeding facilities. RIKEN·SPF mice and SPF mice maintained by Japan SLC, Inc., Charles River Laboratories Japan, Inc., and CLEA Japan, Inc. (hereinafter, SLC·SPF mice, Charles·SPF mice, and Clea·SPF mice) were compared.

As a result, as shown in Fig. 1, regarding the trypsin activity in the cecal contents, RIKEN·SPF mice and SLC·SPF mice were similarly at a low level, and Charles·SPF and Clea·SPF mice were significantly higher than the mice at the former 2 institutions. In addition, although not shown in the figure, regarding the intestinal microbiota, a significant difference was observed on UniFrac-PCoA between the 2 groups of RIKEN·SPF mice and SLC·SPF mice and the 2 groups of Charles·SPF mice and Clea·SPF mice. From the above, it has been found that the difference in the intestinal microbiota due to the difference in the breeding environment affects the trypsin activity beyond the cecum. In addition, it has been suggested that specific intestinal bacterial species may reduce trypsin activity.

### [Intestinal Bacteria of Healthy Individuals Reduce Trypsin Activity in Mice]

The above studies suggest that specific intestinal bacterial species may reduce trypsin activity beyond the cecum, which is suspected to be involved in colon inflammation, in mice. Based on this hypothesis, by colonizing a specific intestinal bacterial species beyond the cecum, trypsin activity can be suppressed, which in turn can be expected to alleviate inflammation of the large intestine. Therefore, assuming future clinical applications, investigation was made on the control of trypsin activity using human-derived intestinal bacteria.

First, the feces of healthy Japanese volunteers (donors A to F) were orally inoculated into the stomach of GF mice to prepare human microbiota-transplanted mice (A to F), and the fecal trypsin activity was evaluated.

As a result, as shown in Fig. 3, the mouse fecal trypsin activity was as low as that in the case of RIKEN·SPF mice, except for B. From this, it has been found that there are human-derived intestinal bacterial species that reduce the activity of mouse trypsin.

### (Example 2)

### [35 Bacterial Strains Derived from Feces of Healthy Individuals Reduce Trypsin Activity in Mouse Feces]

For the feces of healthy volunteer C which had showed particularly low trypsin activity in the above investigation, search was made for bacterial species that reduce trypsin activity beyond the cecum. The feces of healthy volunteer C was orally inoculated into the stomach of GF mice, and 24 hours later, the antibiotics ampicillin (Amp), tylosin, or metronidazole (MNZ) were inoculated by free drinking water.

As a result, as shown in Fig. 4, the fecal trypsin activity was significantly reduced in the antibiotic non-inoculated group and the ampicillin-inoculated group as compared with those before the bacterial inoculation. On the other hand, in the tylosin-inoculated group and the metronidazole-inoculated group, the fecal trypsin activity was maintained high. From the above results, it has been found that the intestinal bacterial species that reduce trypsin activity are resistant to ampicillin and sensitive to tylosin and metronidazole.

Subsequently, the cecal contents of the individual with the lowest trypsin activity value in the ampicillin-inoculated group were cultured in a medium having a different composition in an anaerobic chamber to obtain a total of 432 colonies. As a result of bacterial strain collation based on 16S rRNA analysis, it was revealed that 35 bacterial strains of human intestinal bacteria derived from the feces of donor C had been isolated as shown in Fig. 5.

Since the 35 isolated bacterial strains account for about 80% of the bacterial species constituting the intestinal microbiota of the feces of donor C, it can be expected that a similar microbiota and environment will be formed in the intestine of donor C by inoculation to GF mice. Therefore, a bacterial solution obtained by individually culturing and mixing these 35 bacterial strains (hereinafter, 35-mix) was orally inoculated into the stomach of GF mice, and the trypsin activity beyond the cecum was evaluated over time.

As a result, as shown in Fig. 6, the fecal trypsin activity was significantly decreased 2 days after the inoculation of 35-mix. Even on the 9th day of inoculation, the amount of active trypsin in the feces was maintained at a low level. From the above results, it has been found that inoculation of 35-mix reduces the activity of trypsin beyond the cecum in mice.

### [9 Bacterial Strains Isolated from Feces of Healthy Individuals Reduce Trypsin Activity in Mouse Feces]

Among the above-mentioned 35 bacterial strains having the function of reducing trypsin activity, the responsible bacteria controlling the function were identified. Specifically, in an investigation to colonize GF mice with bacteria derived from the feces of healthy volunteer donor C while inoculating various antibiotics, 12 days after antibiotic inoculation, Spearman's rank correlation analysis of the relative occupancy of intestinal bacteria and the fecal trypsin activity value based on 16S rRNA analysis was performed.

As a result, in the Spearman rank correlation analysis shown in Fig. 7, among the bacterial strains showing a negative correlation with fecal trypsin activity, attention was particularly paid to 9 bacterial strains having a significant probability of correlation coefficient of p < 0.05. Next, a bacterial solution obtained by individually culturing and mixing the 9 bacterial strains (hereinafter, 9-mix) was orally inoculated into the stomach of GF mice, and the activity of trypsin in the feces and the amount of its protein were evaluated. As a result, as shown in Fig. 8, the fecal trypsin activity was significantly reduced in the 9-mix inoculation group. In addition, although not shown in the figure, the amount of active trypsin protein was reduced below the detection limit of WB. From the above results, it has been suggested that the responsible bacteria are contained in the 9 bacterial strains.

### [3 Bacterial Strains Isolated from Feces of Healthy Individuals Reduce Trypsin Activity in Mouse Feces]

As shown in Fig. 9, the above-mentioned 9 bacterial strains can be classified into a group containing 3 Bacteroides bacterial strains and 6 non-Bacteroides bacterial strains. Assuming that the function of reducing the activity of trypsin is systematically conserved, the responsible bacteria are considered to be contained in only one of the groups. In light of the above, in the same manner as in the above investigation, the former Bacteroides-inoculated group (hereinafter, 3-mix inoculation group) and the latter non-Bacteroides-inoculated group (hereinafter, 6-mix inoculation group) were evaluated.

As a result, as shown in Fig. 8, the fecal trypsin activity was significantly decreased in the 3-mix inoculation group, and was not decreased in the 6-mix inoculation group. From the above results, it has been suggested that the responsible bacteria were contained in the above 3 bacterial strains.

### [Paraprevotella Clara Isolated from Feces of Healthy Individuals Reduces Amount of Mouse Trypsin]

For the purpose of further narrowing down the responsible bacteria, trypsin and mono-cultured bacteria were made to coexist in vitro, and changes in the protein amount of trypsin were evaluated.

First, in an anaerobic chamber, His-tag-modified recombinant mouse trypsin and 9-mix, 6-mix, or 3-mix discussed above as a candidate for the responsible bacterium were co-cultured, and the amount of trypsin protein in the culture medium after 12 hours was evaluated.

As a result, although not shown in the figure, a decrease in trypsin was observed in the co-culture with 9-mix or 3-mix, but not in the co-culture with 6-mix. These results are in agreement with the results of the above-mentioned investigation of narrowing down the responsible bacteria in vivo. In other words, this in-vitro evaluation method is considered to be effective in further narrowing down the responsible bacteria. Additionally, the fact that the results of this in-vitro evaluation are in agreement with the results of the above-mentioned in-vivo evaluation suggests that the mechanism of the decrease in trypsin activity does not involve the host colon tissues, but only bacteria.

Subsequently, each bacterium constituting 9-mix was co-cultured with trypsin for each monobacterium and evaluated in the same manner. As a result, as shown in Fig. 10, a decrease in the amount of trypsin protein was observed only when co-cultured with the P. clara 1C4 strain constituting 3-mix.

From the series of evaluation results, it has been found that the P. clara 1C4 strain derived from the feces of donor C is the responsible bacterium for reducing the amount of trypsin protein in mice.

### [Paraprevotella Clara Reduces Amount of Human Trypsin Protein]

As described above, it was demonstrated in vitro that the P. clara 1C4 strain isolated from the feces of a healthy individual reduces the amount of active trypsin in mice. Next, the above-mentioned in vitro experimental system was applied to human trypsin, and the effectiveness of its functions was verified. Specifically, in an anaerobic chamber, a mono-cultured P. clara 1C4 strain and recombinant human trypsin were co-cultured, and the amount of trypsin 12 hours later was evaluated by WB. As a result, although not shown in the figure, a decrease in the amount of human trypsin protein was observed only when co-cultured with the P. clara 1C4 strain. From the above results, it has been found that the P. clara 1C4 strain isolated from the feces of a healthy individual reduces the amount of human trypsin protein.

### (Example 3)

### [Inoculation of Bacterial Cocktail That Reduces Trypsin Activity Suppresses Inflammation of Colon Tissues]

Colitis model mice were used to examine the possibility of alleviating inflammation of the 3 bacterial strains identified (3-mix). For the colitis model mice, an IL10-/- enteritis onset model and a DSS induction model were selected. For the former, the model applied was one in which an IL10-/- mouse individual was made germ-free and then infected with Enterobacter aerogenes 11E12 strain, which was an enteritis-inducing bacterium isolated from the feces of a UC patient.

The 3-mix for reducing trypsin activity or 6-mix not involved in trypsin activity was orally inoculated into the stomach of the IL10-/-GF mouse, and 7 days later, a culture solution of Enterobacter aerogenes 11E12 strain monobacterium was similarly inoculated to induce colitis. The fecal trypsin activity was evaluated up to week 3 after inoculation. In addition, the degree of inflammation induced by week 3 after inoculation was evaluated by the fecal lipocalin level and the inflammation score based on histological observation of the large intestine.

As a result, as shown in Fig. 11, high fecal trypsin activity was observed in the group inoculated with 11E12 strain and the group inoculated with 11E12 strain and 6-mix, while the trypsin activity was suppressed to a low level in the group inoculated with 11E12 strain and the 3-mix. In addition, as shown in Fig. 12, the fecal lipocalin level tended to be lower in the 3-mix inoculation group than in the 6-mix inoculation group. Further, although not shown in the figure, the histological inflammation score was also significantly lower.

A similar investigation was performed on DSS-induced model mice. GF mice were inoculated with 3-mix or 6-mix, and 14 days later, a 2.0% DSS aqueous solution was inoculated by free drinking for 7 days to induce colitis. The degree of inflammation induced by day 10 after DSS inoculation was evaluated by body weight variation, inflammation score based on histological observation of the large intestine, and expression level of inflammation markers. As a result, the rate of weight loss was lower in the 3-mix inoculation group than in the 6-mix inoculation group. In addition, the expression levels of inflammation marker genes including TNF-α and IL6 were also significantly low.

From the above investigation results, it has been found that the inoculation of 3-mix suppresses the inflammation of the colon tissues.

### (Example 4)

### [Genus Paraprevotella Reduces Trypsin Activity]

The preservability of the function of reducing trypsin activity observed in the P. clara 1C4 strain in related bacterial strains was evaluated.

For the P. clara JCM 14859^{T} strain obtained from Japan Collection of Microorganisms, RIKEN BioResource Research Center (RIKEN BRC) and the Paraprevotella xylaniphila (P. xylaniphila) JCM 14860^{T} strain corresponding to the other of the 2 species belonging to the genus Paraprevotella according to the phylogenetic tree classification as of November 2018, the ability to reduce trypsin activity in vitro was verified. A trypsin solution derived from the mouse cecal contents was mixed with a culture solution of each bacterium, incubated under anaerobic conditions for 12 hours, and then subjected to evaluation of the amount of active trypsin protein by WB.

As a result, as shown in Fig. 13, the amount of trypsin protein was also below the detection limit of WB when mixed with any Paraprevotella strain.

From the above results, it has been found that all the bacterial species classified into the genus Paraprevotella in the phylogenetic tree classification as of November 2018 reduce the amount of active trypsin derived from mice.

### (Example 5)

### [Crosslink Test]

As shown in Fig. 14, a band was detected in the vicinity of 250 kDa only when mPRSS2 and the bacteria P. clara were mixed to form a crosslink by DSSO. Since this was not observed in the case of forming a crosslink without containing mPRSS2, it was presumed to be a complex protein composed of mPRSS2 and the constituent proteins of the bacteria P. clara. Considering that mPRSS2 is a protein of about 32 kDa, it is considered that trypsin is preferentially adsorbed or bound to a certain protein of about 220 kDa constituting the bacteria P. clara.

From these results, it is considered that the degradation phenomenon of trypsin protein is induced together with the phenomenon in which trypsin binds to a specific protein possessed by bacteria of the genus Paraprevotella.

### [Trypsin Activity Inhibition Test]

As shown in Fig. 15, when hPRSS2 unmodified with a TLCK inhibitor and bacteria of the genus Paraprevotella were mixed, the hPRSS2-derived band was thinned, and the degradation of hPRSS2 was induced. On the other hand, in the case of TLCK-hPRSS2 having modified TLCK to lose trypsin activity, the degradation of hPRSS2 was not induced. Considering that TLCK is an inhibitor of trypsin activity based on irreversible binding, it is considered that the degradation of trypsin by bacteria of the genus Paraprevotella is due to the promotion of trypsin autolysis.

From the results of the above [Crosslink Test] and [Trypsin Activity Inhibition Test], degradation of trypsin by bacteria of the genus Paraprevotella can be considered to be induced by binding the trypsin protein to a specific protein of the bacteria based on the effect of promoting trypsin autolysis.

### [Industrial Applicability]

As described above, according to the present invention, it is possible to suppress trypsin activity. Therefore, it is useful in treating and preventing diseases caused by trypsin activity (inflammatory bowel diseases such as ulcerative colitis and Crohn's disease).

### [Accession Number]

(1) Identification Label: 1C4
(2) Accession Number: NITE BP-02775
(3) Accession Date: August 30, 2018
(4) Depository Institution: National Institute of Technology and Evaluation
(1) Identification Label: 1D4
(2) Accession Number: NITE BP-02776
(3) Accession Date: August 30, 2018
(4) Depository Institution: National Institute of Technology and Evaluation
(1) Identification Label: 3H3
(2) Accession Number: NITE BP-02777
(3) Accession Date: August 30, 2018
(4) Depository Institution: National Institute of Technology and Evaluation

## Claims

1. A composition for suppressing trypsin activity, comprising: a bacterium belonging to a genus Paraprevotella as an active ingredient.

2. The composition according to claim 1, wherein the bacterium belonging to the genus Paraprevotella is at least one bacterium selected from the group consisting of Paraprevotella clara and Paraprevotella xylaniphila.

3. The composition according to claim 1, wherein the bacterium belonging to the genus Paraprevotella is at least one bacterium having a DNA composed of a base sequence set forth in any of SEQ ID NOs: 1 to 3 or a base sequence having at least 90% identity to the base sequence.

4. The composition according to claim 1, wherein the bacterium belonging to the genus Paraprevotella is at least one bacterial strain selected from the group consisting of a bacterial strain belonging to Paraprevotella clara specified by accession number NITE BP-02775, a Paraprevotella clara JCM 14859^{T} strain, and a Paraprevotella xylaniphila JCM 14860^{T} strain.

5. The composition according to any one of claims 1 to 4, further comprising: at least one bacterium selected from the group consisting of Parabacteroides merdae and Bacteroides uniformis.

6. The composition according to claim 5, wherein the Parabacteroides merdae is at least one bacterium having a DNA composed of a base sequence set forth in SEQ ID NO: 4 or a base sequence having at least 90% identity to the base sequence, and the Bacteroides uniformis is at least one bacterium having a DNA composed of a base sequence set forth in SEQ ID NO: 5 or a base sequence having at least 90% identity to the base sequence.

7. The composition according to claim 5, wherein the Parabacteroides merdae is a bacterial strain belonging to Parabacteroides merdae specified by accession number NITE BP-02776, and the Bacteroides uniformis is a bacterial strain belonging to Bacteroides uniformis specified by accession number NITE BP-02777.

8. A bacterial strain belonging to Paraprevotella clara specified by accession number NITE BP-02775.

9. A bacterial strain belonging to Parabacteroides merdae specified by accession number NITE BP-02776.

10. A bacterial strain belonging to Bacteroides uniformis specified by accession number NITE BP-02777.


SEQUENCE LISTING
<110>  RIKEN
<120>  COMPOSITION FOR SUPPRESSING TRYPSIN ACTIVITY, AND COMPRISING
       BACTERIUM BELONGING TO GENUS PARAPREVOTELLA AS AN ACTIVE
       INGREDIENT
<130>  IBPF19-523WO
<150>  US62/728908
<151>  2018-09-10
<150>  US62/794145
<151>  2019-01-18
<160>  13
<170>  PatentIn version 3.5

<210>  1
<211>  1359
<212>  DNA
<213>  Paraprevotella clara
<220>
<221>  misc_feature
<223>  Paraprevotella clara 1C4
<220>
<221>  misc_feature
<222>  (307)..(308)
<223>  n is a, c, g, or t
<400>  1
cagctttgct gagtttgatg gcgaccggcg cacgggtgag taacgcgtat ccaacctgcc      60
ctttactccg ggatagtctc ctgaaaggga gtttaatacc ggatgtgttt gtttttccgc     120
atgggagcga caaataaaga ttaattggta aaggatgggg atgcgtccca ttagcttgtt     180
ggcggggtaa cggcccacca aggcgacgat gggtaggggt tctgagagga aggtccccca     240
cattggaact gagacacggt ccaaactcct acgggaggca gcagtgagga atattggtca     300
atgggcnnga gcctgaacca gccaagtagc gtgaaggacg acggccctac gggttgtaaa     360
cttcttttat aagggaataa agttcgccac gcgtggtgtt ttgtatgtac cttatgaata     420
agcatcggct aattccgtgc cagcagccgc ggtaatacgg aagatgcgag cgttatccgg     480
atttattggg tttaaaggga gcgtaggcgg gcttttaagt cagcggtcaa atgccacggc     540
tcaaccgtgg ccagccgttg aaactgtaag ccttgagtct gcacagggca catggaattc     600
gtggtgtagc ggtgaaatgc ttagatatca cgaagaactc cgatcgcgaa ggcattgtgc     660
cggggcagca ctgacgctga ggctcgaaag tgcgggtatc aaacaggatt agataccctg     720
gtagtccgca cggtaaacga tgaatgctcg ctatgggcga tatattgtcc gtggccaagc     780
gaaagcgtta agcattccac ctggggagta cgccggcaac ggtgaaactc aaaggaattg     840
acgggggccc gcacaagcgg aggaacatgt ggtttaattc gatgatacgc gaggaacctt     900
acccgggctt gaattgcagg tgcatgagtc agagacggct ctttccttcg ggactcctgt     960
gaaggtgctg catggttgtc gtcagctcgt gccgtgaggt gtcggcttaa gtgccataac    1020
gagcgcaacc cttctcccca gttgccatcg ggtaatgccg ggccctctgg ggacactgcc    1080
atcgtaagat gcgaggaagg tggggatgac gtcaaatcag cacggccctt acgtccgggg    1140
ctacacacgt gttacaatgg ggggtacaga gggccgctgt ccggtgacgg tcggccaatc    1200
cctaaaaccc ctctcagttc ggactggagt ctgcaacccg actccacgaa gctggattcg    1260
ctagtaatcg cgcatcagcc atggcgcggt gaatacgttc ccgggccttg tacacaccgc    1320
ccgtcaagcc atgaaagccg ggggtgcctg aagtccgtg                           1359

<210>  2
<211>  1483
<212>  DNA
<213>  Paraprevotella clara
<220>
<221>  misc_feature
<223>  Paraprevotella clara JCM14859T
<400>  2
gatgaacgct agctacaggc ttaacacatg caagtcgagg ggcagcatgr rcycagcttt      60
gctgggtttg atggcgaccg gcgcacgggt gagtaacgcg tatccaacct gccctttact     120
ccgggatagt ctcctgaaag ggagtttaat accggatgtg tttgtctttc cgcatgggag     180
cgacaaataa agattgattg gtaaaggatg gggatgcgtc ccattagctr gttggcgggg     240
taacggccca ccaaggcrac gatgggtagg ggttctgaga ggaaggtccc ccacattgga     300
actgagacac ggtccaaact cctacgggag gcagcagtga ggaatattgg tcaatgggcg     360

agagcctgaa ccagccaagt agcgtgaagg acgacggccc tacgggttgt aaacttcttt 420
tataagggaa taaagttcgc cacgcgtggt gttttgtatg taccttatga ataagcatcg 480
gctaattccg tgccagcagc cgcggtaata cggaagatgc gagcgttatc cggatttatt 540
gggtttaaag ggagcgtagg cgggctttta agtcagcggt caaatgtcac ggctcaaccg 600
tggccagccg ttgaaactgt aagccttgag tctgcacagg gcacatggaa ttcgtggtgt 660
agcggtgaaa tgcttagata tcacgaagaa ctccgatcgc gaaggcattg tgccggggca 720
gcactgacgc tgaggctcga aagtgcgggt atcaaacagg attagatacc ctggtagtcc 780
gcacggtaaa cgatgaatgc tcgctatggg cgatayawtg tccgtggcca agcgaaagcg 840
ttaagcattc cacctgggga gtacgccggc aacggtgaaa ctcaaaggaa ttgacggggg 900
cccgcacaag cggaggaaca tgtggtttaa ttcgatgata cgcgaggaac cttacccggg 960
cttgaattgc aggtgcatga gtcagagatg attctttcct tcgggactcc tgtgaaggtg 1020
ctgcatggtt gtcgtcagct cgtgccgtga ggtgtcggct taagtgccat aacgagcgca 1080
acccttctcc ccagttgcca tcgggtaatg ccgggccctc tggggacact gccatcgtaa 1140
gatgcgagga aggtggggat gacgtcaaat cagcacggcc cttacgtccg gggctacaca 1200
cgtgttacaa tggggggtac agagggccgc tgtccggtga cggttggcca atccctaaaa 1260
cccctctcag ttcggactgg agtctgcaac ccgactccac gaagctggat tcgctagtaa 1320
tcgcgcatca gccatggcgc ggtgaatacg ttcccgggcc ttgtacacac cgcccgtcaa 1380
gccatgaaag ccgggggtgc ctgaagtccg tgaccgcgag ggtcggccta gggtaaaact 1440
ggtgattggg gctaagtcgt aacaaggtag ccgtaccgga agg 1483

<210> 3
<211> 1481
<212> DNA
<213> Paraprevotella xylaniphila
<220>
<221> misc_feature
<223> Paraprevotella xylaniphila JCM14860T
<400> 3
gatgaacgct agctacaggc ttaacacatg caagtcgagg ggcagcatga acttagcttg 60
ctaagtttga tggcgaccgg cgcacgggtg agtaacgcgt atccaacctg ccctttacgc 120
ggggatagcc ttctgaaagg aagtttaata cccgatgaat tcgtttagtc gcatggcttg 180
atgaataaag atttatcagt aaaggatggg gatgcgtccc attagcttgt tggcggggta 240
acggcccacc aaggcgacga tgggtagggg ttctgagagg aaggtccccc acattggaac 300
tgagacacgg tccaaactcc tacgggaggc agcagtgagg aatattggtc aatgggcgcg 360
agcctgaacc agccaagtag cgtggaggac gacggcccta cgggttgtaa actcctttta 420
taaggggata aagttggcca tgtatggcca tttgcaggta ccttatgaat aagcatcggc 480
taattccgtg ccagcagccg cggtaatacg gaagatgcga gcgttatccg gatttattgg 540
gtttaaaggg agcgtaggcg ggcagtcaag tcagcggtca aatggcgcgg ctcaaccgcg 600
ttccgccgtt gaaactggca gccttgagta tgcacagggt acatggaatt cgtggtgtag 660
cggtgaaatg cttagatatc acgaggaact ccgatcgcgc aggcattgta ccggggcatt 720
actgacgctg aggctcgaag gtgcgggtat caaacaggat tagataccct ggtagtccgc 780
acagtaaacg atgaatgccc gctgtcggcg acatagtgtc ggcggccaag cgaaagcgtt 840
aagcattcca cctggggagt acgccggcaa cggtgaaact caaaggaatt gacgggggcc 900
cgcacaagcg gaggaacatg tggtttaatt cgatgatacg cgaggaacct tacccgggct 960
tgaatcgcag gtgcatgggc cggagacggc cctttccttc gggactcctg cgaaggtgct 1020
gcatggttgt cgtcagctcg tgccgtgagg tgtcggctta agtgccataa cgagcgcaac 1080
ccccctcccc agttgccatc gggtaatgcc gggcactttg gggacactgc caccgcaagg 1140
tgcgaggaag gtggggatga cgtcaaatca gcacggccct tacgtccggg gcgacacacg 1200
tgttacaatg gggggtacag agggccgctg cccggtgacg gttggccaat ccctaaagcc 1260
cctctcagtt cggactggag tctgcaaccc gactccacga agctggattc gctagtaatc 1320
gcgcatcagc catggcgcgg tgaatacgtt cccgggcctt gtacacaccg cccgtcaagc 1380
catgaaagcc gggggtgcct gaagtccgtg accgcgaggg tcggcctagg gtaaaaccgg 1440
tgattggggc taagtcgtaa caaggtagcc gtaccggaag g 1481

<210> 4
<211> 190
<212> DNA
<213> Parabacteroides merdae
<220>
<221> misc_feature
<223> Parabacteroides merdae 1D4
<220>
<221> misc_feature

<222>  (151)..(152)
<223>  n is a, c, g, or t
<400>  4
catgatttgt agcaatacag attgatggcg accggcgcac gggtgagtaa cgcgtatgca      60
acttacctat cagaggggga tagcccggcg aaagtcggat taatacccca taaaacaggg     120
gtcccgcatg ggaatatttg ttaaagattc nncgctgata gataggcatg cgttccatta     180
ggcagttggc                                                            190

<210>  5
<211>  1382
<212>  DNA
<213>  Bacteroides uniformis
<220>
<221>  misc_feature
<223>  Bacteroides uniformis 3H3
<220>
<221>  misc_feature
<222>  (11)..(11)
<223>  n is a, c, g, or t
<220>
<221>  misc_feature
<222>  (176)..(176)
<223>  n is a, c, g, or t
<220>
<221>  misc_feature
<222>  (249)..(250)
<223>  n is a, c, g, or t
<220>
<221>  misc_feature
<222>  (1371)..(1371)
<223>  n is a, c, g, or t
<220>
<221>  misc_feature
<222>  (1374)..(1374)
<223>  n is a, c, g, or t
<400>  5
tcgaggggca ncatgaactt agcttgctaa gtttgatggc gaccggcgca cgggtgagta      60
acacgtatcc aacctgccga tgactcgggg atagcctttc gaaagaaaga ttaatacccg     120
atggcatagt tcttccgcat ggtagaacta ttaaagaact tcggtcatcg atgggnatgc     180
gttccattag gttgttggcg gggtaacggc ccaccaagcc ttcgatggat aggggttctg     240
agaggaagnn cccccacatt ggaactgaga cacggtccaa actcctacgg gaggcagcag     300
tgaggaatat tggtcaatgg acgagagtct gaaccagcca agtagcgtga aggatgactg     360
ccctatgggt tgtaaacttc ttttatacgg gaataaagtg aggcacgtgt gcctttttgt     420
atgtaccgta tgaataagga tcggctaact ccgtgccagc agccgcggta atacggagga     480
tccgagcgtt atccggattt attgggttta aagggagcgt aggcggacgc ttaagtcagt     540
tgtgaaagtt tgcggctcaa ccgtaaaatt gcagttgata ctgggtgtct tgagtacagt     600
agaggcaggc ggaattcgtg gtgtagcggt gaaatgctta gatatcacga agaactccga     660
ttgcgaaggc agcctgctgg actgtaactg acgctgatgc tcgaaagtgt gggtatcaaa     720
caggattaga taccctggta gtccacacag taaacgatga atactcgctg tttgcgatat     780
acagtaagcg gccaagcgaa agcgttaagt attccacctg gggagtacgc cggcaacggt     840
gaaactcaaa ggaattgacg ggggcccgca caagcggagg aacatgtggt ttaattcgat     900
gatacgcgag gaaccttacc cgggcttgaa ttgcaactga atgatgtgga gacatgtcag     960
ccgcaaggca gttgtgaagg tgctgcatgg ttgtcgtcag ctcgtgccgt gaggtgtcgg    1020
cttaagtgcc ataacgagcg caacccttat cgatagttac catcaggtta tgctggggac    1080
tctgtcgaga ctgccgtcgt aagatgtgag gaaggtgggg atgacgtcaa atcagcacgg    1140
cccttacgtc cggggctaca cacgtgttac aatggggggt acagaaggca gctacacggc    1200
gacgtgatgc taatcccgaa agcctctctc agttcggatt ggagtctgca acccgactcc    1260
atgaagctgg attcgctagt aatcgcgcat cagccacggc gcggtgaata cgttcccggg    1320
ccttgtacac accgcccgtc aagccatgaa agccgggggt acctgaagtg ngtnaccgca    1380
ag                                                                   1382

<210>  6
<211>  1376

<212>  DNA
<213>  Paraprevotella clara
<220>
<221>  misc_feature
<223>  Paraprevotella clara 1F6
<220>
<221>  misc_feature
<222>  (31)..(31)
<223>  n is a, c, g, or t
<220>
<221>  misc_feature
<222>  (326)..(327)
<223>  n is a, c, g, or t
<400>  6
tcgaggggca gcatggactc agctttgctg ngtttgatgg cgaccggcgc acgggtgagt      60
aacgcgtatc caacctgccc tttactccgg gatagtctcc tgaaagggag tttaataccg     120
gatgtgtttg tttttccgca tgggagcgac aaataaagat taattggtaa aggatgggga     180
tgcgtcccat tagcttgttg gcggggtaac ggcccaccaa ggcgacgatg ggtaggggtt     240
ctgagaggaa ggtcccccac attggaactg agacacggtc caaactccta cgggaggcag     300
cagtgaggaa tattggtcaa tgggcnngag cctgaaccag ccaagtagcg tgaaggacga     360
cggccctacg ggttgtaaac ttcttttata agggaataaa gttcgccacg cgtggtgttt     420
tgtatgtacc ttatgaataa gcatcggcta attccgtgcc agcagccgcg gtaatacgga     480
agatgcgagc gttatccgga tttattgggt ttaaagggag cgtaggcggg cttttaagtc     540
agcggtcaaa tgccacggct caaccgtggc cagccgttga aactgtaagc cttgagtctg     600
cacagggcac atggaattcg tggtgtagcg gtgaaatgct tagatatcac gaagaactcc     660
gatcgcgaag gcattgtgcc ggggcagcac tgacgctgag gctcgaaagt gcgggtatca     720
aacaggatta gataccctgg tagtccgcac ggtaaacgat gaatgctcgc tatgggcgat     780
atattgtccg tggccaagcg aaagcgttaa gcattccacc tggggagtac gccggcaacg     840
gtgaaactca aaggaattga cgggggcccg cacaagcgga ggaacatgtg gtttaattcg     900
atgatacgcg aggaacctta cccgggcttg aattgcaggt gcatgagtca gagacggctc     960
tttccttcgg gactcctgtg aaggtgctgc atggttgtcg tcagctcgtg ccgtgaggtg    1020
tcggcttaag tgccataacg agcgcaaccc ttctccccag ttgccatcgg gtaatgccgg    1080
gccctctggg gacactgcca tcgtaagatg cgaggaaggt ggggatgacg tcaaatcagc    1140
acggccctta cgtccggggc tacacacgtg ttacaatggg gggtacagag ggccgctgtc    1200
cggtgacggt cggccaatcc ctaaaacccc tctcagttcg gactggagtc tgcaacccga    1260
ctccacgaag ctggattcgc tagtaatcgc gcatcagcca tggcgcggtg aatacgttcc    1320
cgggccttgt acacaccgcc cgtcaagcca tgaaagccgg gggtgcctga agtccg        1376

<210>  7
<211>  1387
<212>  DNA
<213>  Paraprevotella clara
<220>
<221>  misc_feature
<223>  Paraprevotella clara 2C7
<220>
<221>  misc_feature
<222>  (8)..(8)
<223>  n is a, c, g, or t
<220>
<221>  misc_feature
<222>  (321)..(322)
<223>  n is a, c, g, or t
<400>  7
gggcagcntg gactcagctt tgctgagttt gatggcgacc ggcgcacggg tgagtaacgc      60
gtatccaacc tgccctttac tccgggatag tctcctgaaa gggagtttaa taccggatgt     120
gtttgttttt ccgcatggga gcgacaaata aagattaatt ggtaaaggat ggggatgcgt     180
cccattagct tgttggcggg gtaacggccc accaaggcga cgatgggtag gggttctgag     240
aggaaggtcc cccacattgg aactgagaca cggtccaaac tcctacggga ggcagcagtg     300
aggaatattg gtcaatgggc nngagcctga accagccaag tagcgtgaag gacgacggcc     360
ctacgggttg taaacttctt ttataaggga ataaagttcg ccacgcgtgg tgttttgtat     420
gtaccttatg aataagcatc ggctaattcc gtgccagcag ccgcggtaat acggaagatg     480
cgagcgttat ccggatttat tgggtttaaa gggagcgtag gcgggctttt aagtcagcgg     540

tcaaatgcca cggctcaacc gtggccagcc gttgaaactg taagccttga gtctgcacag 600
ggcacatgga attcgtggtg tagcggtgaa atgcttagat atcacgaaga actccgatcg 660
cgaaggcatt gtgccggggc agcactgacg ctgaggctcg aaagtgcggg tatcaaacag 720
gattagatac cctggtagtc cgcacggtaa acgatgaatg ctcgctatgg gcgatatatt 780
gtccgtggcc aagcgaaagc gttaagcatt ccacctgggg agtacgccgg caacggtgaa 840
actcaaagga attgacgggg gcccgcacaa gcggaggaac atgtggttta attcgatgat 900
acgcgaggaa ccttacccgg gcttgaattg caggtgcatg agtcagagac ggctctttcc 960
ttcgggactc ctgtgaaggt gctgcatggt tgtcgtcagc tcgtgccgtg aggtgtcggc 1020
ttaagtgcca taacgagcgc aacccttctc cccagttgcc atcgggtaat gccgggccct 1080
ctggggacac tgccatcgta agatgcgagg aaggtgggga tgacgtcaaa tcagcacggc 1140
ccttacgtcc ggggctacac acgtgttaca atggggggta cagagggccg ctgtccggtg 1200
acggtcggcc aatccctaaa acccctctca gttcggactg gagtctgcaa cccgactcca 1260
cgaagctgga ttcgctagta atcgcgcatc agccatggcg cggtgaatac gttcccgggc 1320
cttgtacaca ccgcccgtca agccatgaaa gccgggggtg cctgaagtcc gtgaccgcga 1380
gggtcgg 1387

<210> 8
<211> 1377
<212> DNA
<213> Paraprevotella clara
<220>
<221> misc_feature
<223> Paraprevotella clara 2D11
<220>
<221> misc_feature
<222> (315)..(316)
<223> n is a, c, g, or t
<400> 8
catggactca gctttgctga gtttgatggc gaccggcgca cgggtgagta acgcgtatcc 60
aacctgccct ttactccggg atagtctcct gaaagggagt ttaataccgg atgtgtttgt 120
ttttccgcat gggagcgaca aataaagatt aattggtaaa ggatggggat gcgtcccatt 180
agcttgttgg cggggtaacg gcccaccaag gcgacgatgg gtaggggttc tgagaggaag 240
gtcccccaca ttggaactga gacacggtcc aaactcctac gggaggcagc agtgaggaat 300
attggtcaat gggcnngagc ctgaaccagc caagtagcgt gaaggacgac ggccctacgg 360
gttgtaaact tcttttataa gggaataaag ttcgccacgc gtggtgtttt gtatgtacct 420
tatgaataag catcggctaa ttccgtgcca gcagccgcgg taatacggaa gatgcgagcg 480
ttatccggat ttattgggtt taaagggagc gtaggcgggc ttttaagtca gcggtcaaat 540
gccacggctc aaccgtggcc agccgttgaa actgtaagcc ttgagtctgc acagggcaca 600
tggaattcgt ggtgtagcgg tgaaatgctt agatatcacg aagaactccg atcgcgaagg 660
cattgtgccg gggcagcact gacgctgagg ctcgaaagtg cgggtatcaa acaggattag 720
ataccctggt agtccgcacg gtaaacgatg aatgctcgct atgggcgata tattgtccgt 780
ggccaagcga aagcgttaag cattccacct ggggagtacg ccggcaacgg tgaaactcaa 840
aggaattgac gggggcccgc acaagcggag gaacatgtgg tttaattcga tgatacgcga 900
ggaaccttac ccgggcttga attgcaggtg catgagtcag agacggctct ttccttcggg 960
actcctgtga aggtgctgca tggttgtcgt cagctcgtgc cgtgaggtgt cggcttaagt 1020
gccataacga gcgcaaccct tctccccagt tgccatcggg taatgccggg ccctctgggg 1080
acactgccat cgtaagatgc gaggaaggtg gggatgacgt caaatcagca cggcccttac 1140
gtccggggct acacacgtgt tacaatgggg ggtacagagg gccgctgtcc ggtgacggtc 1200
ggccaatccc taaaacccct ctcagttcgg actggagtct gcaacccgac tccacgaagc 1260
tggattcgct agtaatcgcg catcagccat ggcgcggtga atacgttccc gggccttgta 1320
cacaccgccc gtcaagccat gaaagccggg ggtgcctgaa gtccgtgacc gcgaggg 1377

<210> 9
<211> 1367
<212> DNA
<213> Paraprevotella clara
<220>
<221> misc_feature
<223> Paraprevotella clara 2G11
<220>
<221> misc_feature
<222> (7)..(7)
<223> n is a, c, g, or t

<220>
<221>  misc_feature
<222>  (19)..(19)
<223>  n is a, c, g, or t
<220>
<221>  misc_feature
<222>  (312)..(312)
<223>  n is a, c, g, or t
<220>
<221>  misc_feature
<222>  (1363)..(1363)
<223>  n is a, c, g, or t
<220>
<221>  misc_feature
<222>  (1366)..(1366)
<223>  n is a, c, g, or t
<400>  9
ggactcngct ttgctgagnt tgatggcgac cggcgcacgg gtgagtaacg cgtatccaac      60
ctgcccttta ctccgggata gtctcctgaa agggagttta ataccggatg tgtttgtttt     120
tccgcatggg agcgacaaat aaagattaat tggtaaagga tggggatgcg tcccattagc     180
ttgttggcgg ggtaacggcc caccaaggcg acgatgggta ggggttctga gaggaaggtc     240
ccccacattg gaactgagac acggtccaaa ctcctacggg aggcagcagt gaggaatatt     300
ggtcaatggg cnggagcctg aaccagccaa gtagcgtgaa ggacgacggc cctacgggtt     360
gtaaacttct tttataaggg aataaagttc gccacgcgtg gtgttttgta tgtaccttat     420
gaataagcat cggctaattc cgtgccagca gccgcggtaa tacggaagat gcgagcgtta     480
tccggattta ttgggtttaa agggagcgta ggcgggcttt taagtcagcg gtcaaatgcc     540
acggctcaac cgtggccagc cgttgaaact gtaagccttg agtctgcaca gggcacatgg     600
aattcgtggt gtagcggtga aatgcttaga tatcacgaag aactccgatc gcgaaggcat     660
tgtgccgggg cagcactgac gctgaggctc gaaagtgcgg gtatcaaaca ggattagata     720
ccctggtagt ccgcacggta aacgatgaat gctcgctatg ggcgatatat tgtccgtggc     780
caagcgaaag cgttaagcat tccacctggg gagtacgccg gcaacggtga aactcaaagg     840
aattgacggg ggcccgcaca agcggaggaa catgtggttt aattcgatga tacgcgagga     900
accttacccg ggcttgaatt gcaggtgcat gagtcagaga cggctctttc cttcgggact     960
cctgtgaagg tgctgcatgg ttgtcgtcag ctcgtgccgt gaggtgtcgg cttaagtgcc    1020
ataacgagcg caacccttct ccccagttgc catcgggtaa tgccgggccc tctggggaca    1080
ctgccatcgt aagatgcgag gaaggtgggg atgacgtcaa atcagcacgg cccttacgtc    1140
cggggctaca cacgtgttac aatggggggt acagagggcc gctgtccggt gacggtcggc    1200
caatccctaa aacccctctc agttcggact ggagtctgca acccgactcc acgaagctgg    1260
attcgctagt aatcgcgcat cagccatggc gcggtgaata cgttcccggg ccttgtacac    1320
accgcccgtc aagccatgaa agccgggggt gcctgaagtc cgnganc                  1367

<210>  10
<211>  29
<212>  DNA
<213>  Artificial Sequence
<220>
<223>  27Forward-mod-1
<400>  10
aatgatacgg cgaccaccga gatctacac                                        29

<210>  11
<211>  53
<212>  DNA
<213>  Artificial Sequence
<220>
<223>  27Forward-mod-2
<400>  11
acactctttc cctacacgac gctcttccga tctagrgttt gatymtggct cag             53

<210>  12
<211>  24
<212>  DNA
<213>  Artificial Sequence

<220>
<223>  338Reverse-1
<400>  12
caagcagaag acggcatacg agat                                              24

<210>  13
<211>  53
<212>  DNA
<213>  Artificial Sequence
<220>
<223>  338Reverse-2
<400>  13
gtgactggag ttcagacgtg tgctcttccg atcttgctgc ctcccgtagg agt              53